(19) Europäisches Patentamt
European Patent Office
Office européen des brevets

(11) EP 1 182 213 A1

(12) **EUROPEAN PATENT APPLICATION**

(43) Date of publication:
**27.02.2002 Bulletin 2002/09**

(21) Application number: **01113996.1**

(22) Date of filing: **08.06.2001**

(51) Int Cl.7: **C07K 19/00**, C07K 14/47, C12N 9/04, C12N 9/16, C12N 9/38, C12N 15/62, G01N 33/50

(84) Designated Contracting States:
**AT BE CH CY DE DK ES FI FR GB GR IE IT LI LU MC NL PT SE TR**
Designated Extension States:
**AL LT LV MK RO SI**

(30) Priority: **12.06.2000 JP 2000174604**
**11.09.2000 JP 2000274219**

(71) Applicant: **Wako Pure Chemical Industries, Ltd.**
**Osaka 540-8605 (JP)**

(72) Inventors:
- **Yamamoto, Sachiko**
**Takatacho Amagasaki, Hyogo 661-0963 (JP)**
- **Shiro, Minoru**
**Takatacho Amagasaki, Hyogo 661-0963 (JP)**
- **Hanada, Toshiro**
**Takatacho Amagasaki, Hyogo 661-0963 (JP)**
- **Kobatake, Shinzo**
**Takatacho Amagasaki, Hyogo 661-0963 (JP)**

(74) Representative:
**Helbing, Jörg, Dr. Dipl.-Chem. et al**
**Patentanwälte von Kreisler-Selting-Werner,**
**Postfach 10 22 41**
**50462 Köln (DE)**

(54) **Hybrid enzyme and use thereof**

(57) Disclosed is a hybrid enzyme containing a foreign peptide, the hybrid enzyme having an enzyme activity similar to that prior to the substitution or insertion of the peptide, and having a property that the hybrid enzyme activity is modulated or modified when a material having binding ability to the peptide introduced by substitution or insertion is bound to the peptide moiety. Using the hybrid enzyme, it becomes possible to assay a trace amount of CRP in a sample by a homogeneous colorimetry, and a macromolecule materials can be easily assayed in a homogeneous system.

EP 1 182 213 A1

**Description**

FIELD OF THE INVENTION

The present invention relates to a hybrid enzyme and a biological substance assay system using the same. More particularly, the present invention relates to a hybrid enzyme containing a part of an amino acid sequence of C-reactive protein (hereinafter briefly referred to as CRP) and CRP assay system using the hybrid enzyme, and a hybrid type of genetically engineered glucose-6-phosphate dehydrogenase (G6PDH) containing an foreign peptide at a specific position and a biological substance assay system using the genetically engineered G6PDH.

BACKGROUND OF THE INVENTION

Biological substance assay systems utilizing the antigen-antibody reaction can be roughly divided into two types: homogeneous assay systems such as turbidimetric immunoassay and latex turbidimetric immunoassay, and heterogeneous assay systems such as an immunoassay using HPLC, electrophoresis etc., radio immunoassay and enzyme immunoassay (EIA). In either case, measurements are made at present using almost automated instruments. However, in the heterogeneous assay systems such as a macromolecule material assays or a small molecule material assays, special instruments are indispensable because solid phases are used. Accordingly, general-purpose (typical) automatic analyzers can not be applied to these systems. Also in the homogeneous assay systems, except for turbidimetric immunoassay and latex turbidimetric immunoassay, special instruments are used, and typical automatic analyzers can not be applied to these systems. On the other hand, the EMIT method (IMMUNOASSAYS, R. M. Nakamura et al., Alan R. Liss, Inc., New York) using an enzyme is established and comes in practice at present as a homogeneous immunoassay system applicable to the typical automatic analyzers. This assay system is a method for measurement of the amount of a material to be assayed (measured) by using an enzyme chemically modified with the material to be assayed and utilizing that enzyme activity is inhibited to result in a reduction in enzyme activity when an antibody to the material to be assayed is bound thereto. However, this system has the problem that the material to be assayed is limited to a small molecule material such as haptene. Then, recently, a homogeneous assay system using a hybrid enzyme formed by inserting a foreign peptide into an enzyme by genic recombination is contrived as a homogeneous immunoassay system which makes it possible to assay a macromolecule material by use of the enzyme. That is to say, this is a method for assaying (measuring) the amount of the foreign peptide, a material containing the peptide in its amino acid sequence, or a material having binding ability to the peptide such as an antibody, by utilizing that enzyme activity can be modulated (modified) when an antibody is bound to a foreign peptide moiety in the hybrid enzyme. As the enzymes used in this method, there are known alkaline phosphatases (Published Japanese Translation of PCT International Publication (Toku-Hyo-Hei) 8-507686 (1996)) and β-galactosidases (FEBS Letters, 434, 23-27 (1998), FEBS Letters, 438, 267-271 (1998)). In this method, however, it is also known that the hybrid enzyme sometimes loses enzyme activity according to the position where a foreign peptide is inserted, or that even when the hybrid enzyme has enzyme activity, the enzyme activity sometimes can not be modulated (modified) when the material having binding ability is bound to the hybrid enzyme.

Meanwhile, CRP, which is one of major plasma proteins which reacts with C polysaccharide of Streptococcus Pneumoniae, is known to be acute phase reactant protein and to increase in an inflammation or tissue injury. The measurement of CRP is useful for early diagnosis of inflammation in tissue and diagnosis of diseases. Conventionally, CRP has been measured by turbidimetric immunoassay, latex turbidimetric immunoassay, enzyme immunoassay or radio immunoassay. Of these, turbidimetric immunoassay and latex turbidimetric immunoassay are applicable to the typical automatic analyzers. However, as both are a method for detecting changes in turbidity, they tend to be effected by factors of instruments and have a problem with regard to accuracy on measurement. Accordingly, it has been desired to develop homogeneous immunoassay systems using enzymes for measuring biological substances such as CRP and applying to the typical automatic analyzers.

SUMMARY OF THE INVENTION

In view of the situation as described above, an object of the present invention is to provide a method which makes it possible to assay a trace amount of CRP in a sample by a homogeneous colorimetry. It is another object of the present invention to provide a method for measuring a macromolecule material in a homogeneous system using a enzyme.

As a result of intensive research for attaining the above-mentioned objects, the present inventors have found that the use of the above-mentioned hybrid enzyme (i.e. the genetically engineered enzyme) makes it possible to assay (measure) in a homogeneous system a macromolecule material to be assayed (measured), and have considered to be capable of developing a new CRP measuring method applicable to the typical automatic analyzers by application of this method to the assay of CRP, because the detection thereof can be conducted by a colorimetry. The present inventors have conducted further research, and have discovered that the above-mentioned objects can be attained by using a hybrid enzyme containing a part of a material to be analyzed such as CRP at a certain position of some kind of enzyme, thus completing the present invention.

That is to say, as an effective hybrid enzyme for providing a method which makes it possible to measure a trace amount of CRP in a sample by a homogeneous colorimetry using a hybrid enzyme in which a CRP-derived peptide is inserted into a specific position, the present invention provides (1) a hybrid enzyme which has a partial substitution or an insertion of a peptide containing a part of an amino acid sequence represented by SEQ ID NO:1, in which said hybrid enzyme has the same enzyme activity as an original enzyme without the substitution or the insertion of said peptide, and said hybrid enzyme activity is modulated when a material having binding ability to said peptide introduced by the substitution or the insertion is bound to the peptide moiety; (2) the hybrid enzyme described in (1), in which the peptide comprises an amino acid sequence having at least 6 or more sequential amino acid residues selected from the amino acid sequence of SEQ ID NO: 1; (3) the hybrid enzyme described in (2), in which the peptide has a property of being capable of binding to a material having binding ability to CRP; (4) the hybrid enzyme described in (1), in which the peptide comprises an amino acid sequence having at least 6 or more sequential amino acid residues selected from any one of SEQ ID NO: 2 through SEQ ID NO: 5; (5) the hybrid enzyme described in (1), in which the original enzyme is a glucose-6-phosphate dehydrogenase (hereinafter sometimes referred to as G6PDH), a β-galactosidase or an alkaline phosphatase; and (6) the hybrid enzyme described in (1), in which the material having binding ability to the peptide is an antibody. As a method for measuring a trace amount of CRP in a sample by a homogeneous colorimetry using the above-mentioned hybrid enzyme, the present invention provides (7) a reagent for measurement of CPR comprising the hybrid enzyme described in any one of (1) through (6); (8) the reagent described in (7) further comprising an anti-CRP antibody; (9) a kit for measurement of CRP containing a reagent comprising the hybrid enzyme described in any one of (1) through (6); (10) the kit described in (9) further comprising an anti-CRP antibody; (11) a method for measurement of CRP which is characterized in using the hybrid enzyme described in any one of (1) through (6); (12) the method described in (11) further comprising using an anti-CRP antibody in combination; and (13) a method for measurement of CRP comprising bringing a sample containing CRP, the hybrid enzyme described in any one of (1) through (6) and an anti-CRP antibody into contact with one another, then determining an activity of the hybrid enzyme, and determining the amount of CRP in the sample based on the resulting enzyme activity.

Further, as a G6PDH-containing hybrid enzyme having a similar enzyme activity also when a foreign peptide is inserted therein, and having a property that the hybrid enzyme activity can be modulated when a material having binding ability to the foreign peptide moiety is bound thereto, for measurement of a macromolecule material in a homogeneous system, the present invention provides (14) a hybrid enzyme having a peptide introduced into a specific position of a G6PDH by insertion or substitution; (15) the hybrid enzyme described in (14), in which the specific position is a position at which the G6PDH activity can be maintained even in the insertion or substitution of a peptide having 6 or more amino acid residues; (16) the hybrid enzyme described in (14), in which the specific position is a position at which the G6PDH activity is modulated when a material having binding ability to the peptide introduced by insertion or substitution is bound to the peptide; (17) the hybrid enzyme described in (14), in which the specific position is any position selected from the group consisting of the position between 294-295(hereinafter sometimes refered to as asp294/ser295 or Asp294 position), between 302-310, between 362-363, the N-terminal and the C-terminal of the amino acid sequence of G6PDH represented by SEQ ID NO: 6; (18) the hybrid enzyme described in (14), in which the peptide is selected from the amino acid sequence of CRP; and (19) the hybrid enzyme described in (14), in which the peptide has a character that there is a material having binding ability specifically to the part of the hybrid enzyme in which the peptide is substituted or inserted. The present invention further provides (20) a reagent comprising the hybrid enzyme described in any one of (14) through (19), for measurement of a material containing the peptide introduced into the hybrid enzyme according to any one of (14) through (19) by insertion or substitution; (21) a kit comprising the hybrid enzyme described in any one of (14) through (19), for measurement of a material containing the peptide introduced into the hybrid enzyme according to any one of (14) through (19) by insertion or substitution; (22) a method comprising using the hybrid enzyme described in any one of (14) through (19), for measurement of a material containing the peptide introduced into the hybrid enzyme according to any one of (14) through (19) by insertion or substitution; (23) a method comprising using the hybrid enzyme described in any one of (14) through (19) in combination with a material having binding ability to the peptide introduced into the hybrid enzyme according to any one of (14) through (19) by insertion or substitution, for measurement of a material containing the peptide; (24) a method for measurement of a material containing said peptide introduced into the hybrid enzyme according to any one of (14) through (19), which comprises bringing the hybrid enzyme according to any one of (14) through (19), a sample containing a material containing the peptide introduced into said hybrid enzyme by insertion or substitution and a material having binding ability to said peptide into contact with one another, then measuring activity of said hybrid enzyme, and determining the amount of the material containing said peptide in the sample based on the resulting enzyme activity; (25) a reagent comprising the hybrid enzyme described in any one of (14) through (19), for measurement of a material having binding ability to the peptide introduced into the hybrid enzyme according to any one of (14) through (19) by insertion or substitution; (26) a kit comprising the hybrid enzyme described in any one of (14) through (19), for measurement of a material having binding ability to the peptide introduced into the hybrid enzyme according to any one of (14) through (19) by insertion or substitution; (27) a method comprising using the hybrid enzyme described in any one of (14) through (19), for measurement of a material having binding ability to the peptide introduced into the hybrid enzyme according to any one of (14) through (19) by insertion or substitution; and (28) a method for measurement of a material having binding ability to said peptide introduced into the hybrid enzyme according to any one of (14) through (19), which comprises bringing the hybrid enzyme according to any one of (14) through (19) into contact with a sample containing a material having binding ability to the peptide, then measuring an activity of said hybrid enzyme, and determining the amount of the material having binding ability to said peptide in the sample based on the resulting enzyme activity.

In the present invention, in order to produce a reagent for detecting a certain material or a material having binding ability thereto, it has been discovered that (29) a gene coding for a hybrid enzyme comprising an amino acid sequence into which a foreign peptide is introduced by substitution or insertion at any position selected from the group consisting of the position between 294-295, between 302-310, between 362-363, the N-terminal and the C-terminal of the amino acid sequence of G6PDH represented by SEQ ID NO: 6 is useful. The present invention further provides (30) a recombinant DNA which is characterized in inserting the hybrid enzyme gene described in (29) into a vector DNA; (31) a transformant or a transductant comprising the recombinant DNA described in (30); (32) a method for producing a protein having an enzyme activity of G6PDH and a property that the G6PDH activity is modulated when a material having binding ability to an amino acid sequence introduced into G6PDH by substitution or insertion is bound to the amino acid sequence, which comprises cultivating the transformant or the transductant described in (31), and collecting the protein; (33) a gene coding for a hybrid enzyme comprising an amino acid sequence into which an amino acid sequence , which can be cleaved with a restriction enzyme is introduced by substitution or insertion at any position selected from the group consisting of the Asp294 position, the Leu302 through Asp310 positions, the Glu362 position, the N-terminal and the C-terminal of the amino acid sequence of G6PDH represented by SEQ ID NO: 6; and (34) a recombinant DNA which is characterized in inserting the hybrid enzyme gene described in (33) a vector DNA.

Still further, the present invention provides (35) a hybrid enzyme in which a peptide selected from an amino acid sequence represented by SEQ ID NO: 1 is introduced into a specific position of a β-galactosidase by insertion or substitution; (36) the hybrid enzyme described in (35), in which the specific position is a site selected from the position between 280-281 and between796-797 of an amino acid sequence of a β-galactosidase represented by SEQ ID NO: 30; (37) a gene coding for the hybrid enzyme described in (36); (38) a recombinant DNA, which is characterized in inserting the hybrid enzyme gene described in (37) into a vector DNA; (39) a transformant or a transductant comprising the recombinant DNA described in (38); and (40) a method for producing a protein having an enzyme activity of a β-galactosidase and a property that the β-galactosidase activity is modulated when a material having binding ability to an amino acid sequence introduced into the β-galactosidase by substitution or insertion is bound to the amino acid sequence, which comprises cultivating the transformant or the transductant described in (39), and collecting the protein.

Furthermore, the present invention provides (41) a hybrid enzyme in which a peptide selected from an amino acid sequence represented by SEQ ID NO: 1 is introduced into a specific position of an alkaline phosphatase by insertion or substitution; (42) the hybrid enzyme described in (41), in which the specific position is a site selected from the position between 167-168, between 168-169, btween 407-408, between 91-93 and between 169-177 of an amino acid sequence of an alkaline phosphatase represented by SEQ ID NO: 31; (43) a gene coding for the hybrid enzyme described in (42); (44) a novel recombinant DNA , which is characterized in inserting the hybrid enzyme gene described in (43) into a vector DNA; (45) a transformant or a transductant comprising the recombinant DNA described in (44); and (46) a method for producing a protein having an enzyme activity of an alkaline phosphatase and having a property that the alkaline phosphatase activity is modulated when a material having binding ability to an amino acid sequence introduced into the alkaline phosphatase by substitution or insertion is bound to the amino acid sequence, which comprises cultivating the transformant or the transductant described in (45), and collecting the protein.

## BRIEF DESCRIPTION OF THE DRAWINGS

Fig. 1 is a graph showing the ability for measuring an anti-CRP antibody of hybrid enzyme G308C1 of the present invention;

Fig. 2 is a graph showing the ability for measuring CRP of hybrid enzyme G308C1 of the present invention. In Figs. 2 to 14,

Recovery (%) = {(G6PDH activity at each CRP concentration) - (G6PDH

activity at 0 mg/dl of CRP)}/{(G6PDH activity in the absence of antibody)

-(G6PDH activity at 0 mg/dl of CRP)} X 100;

and

Activity ratio (%)= {G6PDH activity in the presence of antibody/G6PDH activity in the absence of antibody} X 100;

Fig. 3 is a graph showing the ability for measuring CRP of hybrid enzyme G308C2 of the present invention;
Fig. 4 is a graph showing the ability for measuring CRP of hybrid enzyme G308C3 of the present invention;
Fig. 5 is a graph showing the ability for measuring CRP of hybrid enzyme G308C5 of the present invention;
Fig. 6 is a graph showing the ability for measuring CRP of hybrid enzyme G306C1 of the present invention;
Fig. 7 is a graph showing the ability for measuring CRP of hybrid enzyme G309C1 of the present invention;
Fig. 8 is a graph showing the ability for measuring the anti-CRP of hybrid enzyme G362C1 of the present invention;
Fig. 9 is a graph comparing the sensitivities of measuring CRP of hybrid enzymes G306C1, G306C15 and G306C18 of the present invention;
Fig. 10 is a graph showing the ability for measuring CRP of hybrid enzyme G306d3C1 of the present invention;
Fig. 11 is a graph showing the ability for measuring the anti-preS2 antibody of hybrid enzyme G306H1 of the present invention;
Fig. 12 is a graph showing the ability for measuring the PTH of hybrid enzyme G306P1 of the present invention;
Fig. 13 is a graph showing the ability for measuring the anti-CRP of hybrid enzyme B796C1 of the present invention; and
Fig. 14 is a graph showing the ability for measuring CRP of hybrid enzyme B796C1 of the present invention.

## DESCRIPTION OF THE PREFERRED EMBODIMENTS

Origin enzymes used for the hybrid enzymes of the present invention may be any, as long as they are enzymes generally used. Examples thereof include adenosine deaminases, alkaline phosphatases, α-amylases, bacterial luciferases, β-galactosidases, β-galactosidase fragments, β-lactamases, carbonic anhydrases, catalases, firefly luciferases, glucose oxidases, glucose-6-phosphate dehydrogenases, glucosidases, hexokinases, horseradish peroxidases, invertases, isocitrate dehydrogenases, lysozymes, malate dehydrogenases, micro peroxidases, 6-phosphofructases and xanthine oxidases.

Further, all enzymes can be used, as long as each amino acid sequence or DNA sequence arrangement thereof is determinate or can be determined, and the enzyme activity can be modulated when a part or plural parts of the enzyme are substituted by a foreign peptide or a foreign peptide is inserted into a part or plural parts thereof and a material having binding ability to the peptide is bound to the peptide. Among which, enzymes having high enzyme activity, ones having good stability and ones which can be assayed by the colorimetry are particularly preferred. Examples thereof include G6PDHs, β-galactosidases and alkaline phosphatases. Genes of these enzymes are available by cloning methods from genomes usually employed. Of course, already cloned genes or synthetic DNA can also be used. These enzymes may be derived from any, and each amino acid sequence thereof may be a sequence obtained by deletion, substitution or addition of one or more amino acids, as long as they have intrinsic enzyme activity.

The foreign peptide used for preparing the hybrid enzyme of the present invention may be any, as long as a material such as an antibody or a receptor having binding ability to the peptide is present. Donors thereof include, for example, biological substances such as CRP, IgG, IgA, IgM, C3, C4, β2 microglobulin and albumin, various cancer markers such as α-fetoprotein, CA19-9, prostatic specific antigen (PSA) and carcinoembryonic antigen (CEA), various hormones such as insulin, human chorionic gonadotropin (hCG), prolactin, parathyroid hormone and thyroid stimulating hormone (TSH), various toxins such as streptolysin 0 (SL0), or various viruses such as hepatitis B virus (HBV), hepatitis C virus (HCV), human immunodeficiency virus (HIV) and human papilloma virus (HPV), which are generally assayed (measured) in immunoassay systems.

There is no particular limitation on the vector used in the recombinant DNA of the present invention, as long as it is a vector such as a plasmid vector and a bacterio phage λ vector, which can be replicated and inherited in various hosts of procaryotic cells and/or eucaryotic cells. Examples of such vectors include Escherichia coli (E coli)-derived plasmids such as pBR322, pBR325, pUC12, pUC13 and pBluescript, yeast-derived plasmids such as pSH19 and pSH15, and Bacillus subtilis-derived plasmids such as pUB110, pTP5 and pC194, which are generally available in this field. Further, examples of the bacterio phage λ vectors include bacteriophages such as λ phage, and viruses such as retroviruses, vaccinia viruses and nuclear polyhedrosis viruses.

The host cells used for the production of the hybrid enzymes of the present invention include bacteria (for example, E. coli), yeast (for example, Saccharomyces), animal cells (for example, chinese hamster cell CHO) and insect cells (for example, BmN4).

Construction of recombinant DNA, and expression and purification of the hybrid enzyme for producing the hybrid enzymes of the present invention may be carried out by known methods, for example, methods described in Molecular Cloning (J. Sambrook et al., 2nd Edition, Cold Spring Harbor Laboratory).

There is no particular limitation on an expression vector, as long as it can be replicated and inherited in various hosts of procaryotic cells and/or eucaryotic cells, and has the function of expressing genes the hybrid enzymes from cloned gene in various hosts of procaryotic cells and/or eucaryotic cells, that is to say, the function of producing the desired hybrid enzymes. For example, the vectors generally available in this field are preferably pBR322, pUC12, pUC13, pTrcHis, pTrc99A, pMAL-c2 and artificially modified products thereof (DNA fragments obtained by treating the vectors with appropriate restriction enzymes) when the host cell is E. coli; pRS403, pRS404, pRS413, pRS414 and pYES2 when the host cell is yeast; plasmids pRSVneo ATCC37224, pSV2dhfr ATCC37145, pdBPV-MMTneo ATCC37224 and pSV2neo ATCC37149 when the host cell is an animal cell; and Autographica california nuclear polyhedrosis virus (AcNPV) and Bombyx mori nuclear polyhedrosis virus (BmNPV) when the host cell is an insect cell.

In the following, the preparation of the hybrid enzyme of the present invention is described as to the case that G6PDH is used as the original enzyme, a CRP-derived peptide as the foreign peptide, and E. coli as the host cell.

The G6PDH gene can be obtained, for example, by the following method. That is to say, first, cells are harvested from a culture product of Leuconostoc mesenteroides by centrifugation according to a conventional method described in Molecular Cloning (J. Sambrook et al., 2nd Edition, Cold Spring Harbor Laboratory), and genomic DNA is extracted. Operations are hereinafter conducted according to well-known techniques generally employed, which are described in Molecular Cloning (J. Sambrook et al., 2nd Edition, Cold Spring Harbor Laboratory) and the like, unless otherwise specified. Using the above-mentioned genomic DNA as a template, an oligonucleotide primer having an N-terminal sequence and a C-terminal sequence of the G6PDH gene sequence shown in R. Levy et al., J. Biological Chemistry, 266, 13028- (1991), or a sequence upstream therefrom and a sequence downstream therefrom is added, and the polymerase chain reaction is conducted by using a DNA thermal cycler (Perkin Elmer) to specifically amplify a DNA fragment containing the G6PDH gene. The resulting DNA fragment is integrated into a vector DNA according to a conventional method, thereby obtaining recombinant DNA containing DNA coding for G6PDH.

Further, for connecting the foreign peptide to the G6PDH gene by insertion or substitution, any method may be used. For example, the method for preparing a deletion mutant using- an exonuclease described in "Laboratory Manual Genetic Engineering" (edited by Seikan Muramatsu, the 3rd edition, pages 219 to 230, Maruzen), the method for introducing artificial mutation such as the Kunkel method, the cassette method or the method using the PCR, or the phosphorothioate method described in "DNA Cloning 1" (edited by D.M. Glover et al., 2nd Edition, pages 197 to 228, Takara), the gap double stranded DNA method and the MHT protocol can be used in combination with each other. The resulting DNA coding for the hybrid enzyme is normally inserted into the vector DNA according to conventional method, thereby being able to obtain the recombinant DNA containing the DNA coding for the hybrid enzyme. Then, a method for preparing a restriction enzyme site in the G6PDH gene inserted into the plasmid, and connecting the foreign peptide by insertion or substitution is described below.

That is to say, using the G6PDH gene as a template, primers to which a site cleavable with a restriction enzyme such as BamH I (BamH I site) is added in order to insert the BamH I site into the 5'-terminal of base sequences coding for amino acid sequences on both sides of a insertion position of a foreign peptide in G6PDH, is subjected to the PCR, in combination with primers of base sequences coding for upstream part or downstream part from a position where a foreign peptide is to be inserted respectively, to amplify DNA fragments to which the BamH I site is inserted. In other word, using the G6PDH gene as a template, oligonucleotide containing a sequence of a sense strand downstream from the position to be inserted in the G6PDH gene and a recognition sequence for a restriction enzyme(BamH I site) at the 5'-terminal to be inserted and oligonucleotide containing an anti-sense strand sequence of C-terminal in the G6PDH gene in combination, and oligonucleotide containing a sequence of an anti-sense strand upstream from the position to be inserted in the G6PDH gene and BamH I site at the 5'-terminal to be inserted and oligonucleotide containing an sense strand sequence of N-terminal in the G6PDH gene in combination, are used to amplify DNA fragments to which a BamH I site is added.

Then, two kinds of fragments thus obtained are connected to each other on the vector, thereby being able to construct the G6PDH gene into which the BamH I site is inserted. The restriction enzyme site is cleaved with the restriction enzyme, thereby being able to insert any DNA fragment having a complementary sequence and the cleaved site at both ends thereof.

When any amino acid sequence is eliminated for inserting the peptide by substitution, both sides of the sequence to be eliminated are subjected to the same operation as described above. Thus, the G6PDH gene connected to the foreign peptide by substitution can be obtained.

When the recombinant DNA is constructed, secretory production can also be conducted as a fused protein of the hybrid enzyme and another protein or peptide. Further, the fused protein produced by secretion can also be cleaved with an appropriate protease or by chemical treatment to obtain the hybrid enzyme. Examples of the proteins to be fused include maltose binding proteins and glutathione S-transferase, and examples of the peptides to be fused include histidine tags and FLAG tags.

The basic vectors for the construction of the recombinant DNA include, for example, plasmid vectors such as pBR322 (J. G. Sutcliffe, Cold Spring Harbor Symposium, 43, 77 (1979)), pUC18/19 (C. Yanisch-Perron et al., Gene, 33, 102-119 (1985)), pBluescript IISK+ (STRATEGENE), pMAL-C2 (NEW England Biolabs), pTrc99A (Amersham Pharmacia), pKK223-3 (Amersham Pharmacia) and pET-11 (STRATEGENE), and bacteriophage vectors such as λENBL3 (STRATEGENE) and λDASHII (Funakoshi).

A promoter used in the recombinant DNA may be any, as long as it functions in E. coli. Examples thereof include a lac promoter, a trp promoter, a T7 promoter and derivatives thereof. Further, the recombinant DNA may contain an initiation signal such as a liposome binding sequence functioning in E. coli and a terminator. It may further contain a selective marker gene such as an ampicillin-resistance gene or a tetracycline--resistant gene.

Using the recombinant DNA thus constructed, E. coli is transformed or transduced to prepare a transformant or a transductant. E. coli includes M103, JA221, HB101, C600, XL1-Blue and JM109.

Methods for transforming or transducing the recombinant DNA into E. coli include, for example, the method of Cohen et al. (Proc. Natl. Acad. Sci. U.S.A., 69, 2110 (1972)) and the method of Hanahan et al. (J. Mol. Biol., 166, 557 (1983)). The recombinant DNA may be obtained from the recombinant DNA-containing transformant or transductant by a conventional method, for example, the method of Birnborin et al. (Nucic Acid Res 7, 1513(1979)).

The hybrid enzyme of the present invention can be produced by cultivating the transformant or transductant of the recombinant DNA prepared as described above. Media used include, for example, Luria-Bertani medium (Molecular Cloning (J. Sambrook et al., 2nd Edition, Cold Spring Harbor Laboratory)), 2×YT medium (Molecular Cloning (J. Sambrook et al., 2nd Edition, Cold Spring Harbor Laboratory)) and M9 medium (J. Miller, Exp. Mol. Genet., Cold Spring Harbor Laboratory, New York, page 431 (1972)). The pH of the medium is preferably from 5 to 8. , The cultivation is conducted usually at 14 to 42°C, preferably at 28 to 39°C, for 3 to 24 hours, optionally with aeration or stirring. Further, an expression inducing reagent such as isopropyl-β-D-1-thiogalactopyranoside, or an antibiotic such as ampicillin or chloramphenicol may be added as needed.

The hybrid enzyme of the present invention can be obtained in the following manner from the culture product obtained by the above-mentioned cultivation. That is to say, when the hybrid enzyme exists in a culture solution of the culture product, a culture filtrate or culture supernatant containing the hybrid enzyme is obtained by a conventional method such as filtration or centrifugation. On the other hand, when the hybrid enzyme exists in periplasms or cells of the cultivated transformant or transductant, the culture product is subjected to a conventional method such as filtration or centrifugation to collect the periplasms or cells, which is suspended in a proper buffer solution and the cells in this solution are disrupted by a conventional method such as ultrasonication, lysozyme treatment or freeze-thawing. Then, a crude extract solution containing the hybrid enzyme is obtained by a conventional method such as filtration or centrifugation.

The hybrid enzyme may be separated and purified from the thus obtained culture filtrate, culture supernatant or crude extract solution containing the hybrid enzyme of the present invention by a suitable combination of known separating and purifying methods. These known separating and purifying methods include methods mainly utilizing a difference in solubility such as salting-out and salt precipitation, methods mainly utilizing a difference in molecular weight such as dialysis, ultrafiltration, gel filtration and SDS-polyacrylamide gel electrophoresis, methods utilizing a difference in electric charge such as ion exchange chromatography, methods utilizing a difference in hydrophobicity such as hydrophobic chromatography, methods utilizing a difference in isoelectric point such as isoelectric point electrophoresis, and methods utilizing specific affinity such as affinity chromatography.

The effect of the anti-CRP antibody to the enzyme activity of the resulting hybrid enzyme can be examined, for example, in the following manner. That is to say, the enzyme activity of the hybrid enzyme is assayed(measured) in the absence and presence of the anti-CRP antibody, respectively, to examine modulations in activity due to the binding of the anti-CRP antibody.

To 6 µl of a solution obtained by diluting each hybrid enzyme solution with 100 mM Tris/HCl buffer (pH 7.8) containing 1% bovine albumin, 3 mM magnesium chloride and 150 mM sodium chloride to about 1 U/ml, 150 µl of 100 mM Tris/HCl buffer containing 3 mM magnesium chloride and 150 mM sodium chloride (pH 7.8, hereinafter referred to as buffer A for brevity), or an antibody solution obtained by diluting 100 times anti-CPR goat antibody with buffer A is added, followed by reaction at 37°C for 5 minutes. Then, 75 µl of buffer A containing 10 mM glucose-6-phosphate (G6P) and 6 mM nicotinamide adenine dinucleotide (NAD) are added thereto, followed by reaction at 37°C for 5 minutes, and the changes in absorbance at a wavelength of 340 nm for 5 minutes are determined as G6PDH activity.

The foreign peptide inserted into the enzyme may be any, as long as it maintains the structure that a material having binding ability to the peptide can bind thereto, and its enzyme activity is maintained even by insertion of the peptide. However, as described in Antibodies A Laboratory Manual (Ed Harlow et al., pages 76-, Cold Spring Harbor Laboratory), the peptide to be inserted is required to have at least 6 or more sequential amino acid residues for maintaining its antigenicity. When the foreign peptide to be inserted is a CRP-derived peptide, all or a part of the whole amino acid sequence of CRP represented by SEQ ID NO: 1 is used. Of these, an amino acid sequence containing 6 to 50 sequential amino acid residues, for example, a sequential amino acid sequence selected from Gln(1)-Asp(16), Glu(14)-Ala(24), Leu(22)-Ser(45), Thr(41)-Asn(61), Arg(47)-Ile(63), Lys(114)-Lys(121), Glu(130)-Glu(138), Ile(134) -Gly(148), Gln(137)-Leu(152), Glu(147)-Leu(152), Asp(3)-Ser(18), Leu(152)-Val(165), Val(165)-Gly(178), Leu(121) -Ser(132) and Arg(188)-Glu(197), is preferred. In particular, an amino acid sequence containing at least 6 or more sequential amino acid residues selected from sequences represented by SEQ ID NO: 2 (Asp(3)-Ser(18)), SEQ ID NO: 3 (Leu(152)-Gly(178)), SEQ ID NO: 4 (Leu(121)-Ser(132)) and SEQ ID NO: 5 (Arg(188)-Glu(197)) is more preferred. The peptide moiety introduced into the enzyme by insertion or substitution may contain a sequence other than the desired peptide, such as a restriction enzyme site, which is sometimes introduced in the course of preparing the hybrid enzyme.

In the present invention, the foreign peptide may be inserted into the origin enzyme of the hybrid enzyme at any position, as long as the enzyme activity is maintained also when the foreign peptide is inserted, and is modulated (modified) when a material having binding ability to the inserted foreign peptide is bound to the peptide. The positions considered to be suitable therefor include a site exposed on a surface of the enzyme and a site where activation is influenced. The preferred position at which the foreign peptide is inserted into G6PDH as the origin enzyme was studied using the CRP-derived peptide. That is to say, we tried to insert the foreign peptide into the N-terminal, 32/33 (which means "between the 32nd and the 33rd", hereinafter the same), 37/38, 48/49, 66/67, 87/88, 139/140, 226/227, 294/295, 296/297, 302/303, 305-310, 362/363, 409/410 and the C-terminal of G6PDH. Results thereof show that the effective sites for CRP measurement at which the enzyme activity remains in the insertion of the peptide and is modulated by the anti-CRP antibody reaction are the N-terminal, 294/295, 302/303, 305/306, 306/307, 308/309, 309/310, 362/363 and the C-terminal, as shown in Table 1.

Table 1

| Insertion Site | Activity in Peptide Insertion | Modulations in Enzyme Activity in Antibody Reaction |
|---|---|---|
| Lys32/Lys33 | - | - |
| Gln37/Lys38 | - | - |
| Gln48/Ala49 | + | - |
| Phe66/Thr67 | + | - |
| Va187/Thr88 | - | - |
| Gly226/Tyr227 | - | - |
| Asp294/Ser295 | + | + |
| Ala296/Asp297 | - | - |
| Leu305/Asp306 | + | + |
| Asp306/Va1307 | + | + |
| Pro308/Ala309 | + | + |
| Ala309/Asp310 | + | + |
| Glu329/Gly330 | - | - |
| Glu362/Gln363 | + | + |
| Lys409/Lys410 | - | - |
| C-terminal | + | + |

The partial substitution of the amino acid sequence of the enzyme by the foreign peptide means that amino acid residues (an amino acid sequence) at specific sites (portions) of the enzyme are substituted by the amino acid sequence of the foreign peptide. In this case, the amino acid residue of the peptide introduced by substitution may be either more or less than the amino acid residues eliminated from the enzyme, as long as the enzyme activity of the original enzyme is maintained also after substitution, and is modulated when a material having binding ability to the foreign peptide introduced by substitution is bound to the peptide. It is preferred that the number of the amino acid residues eliminated is approximately the same as that of the amino acid residues introduced. Further, it is preferred that the number of the amino acid residues of the peptide introduced by substitution is approximately the same as that of the amino acid residues of the foreign peptide introduced into the above-mentioned enzyme. Still further, the origin enzyme may be substituted by the foreign peptide at any position, as long as the enzyme activity is maintained also after substitution, and is modulated when a material having binding ability to the foreign peptide introduced by substitution is bound to the peptide. The position at which the foreign peptide is introduced by substitution is selected, based on the position at which the foreign peptide can be inserted into the original enzyme of the hybrid enzyme, as shown above.

In the present invention, the hybrid enzyme obtained as described above is used for qualitative analysis or quantitative analysis

The hybrid enzyme of the present invention is modulated in its enzyme activity according to the binding amount thereof, when a material having binding ability to the foreign peptide introduced by substitution or insertion is bound to the peptide. Accordingly, the presence and amount of the material having the binding ability to the foreign peptide can be detected by allowing a sample containing the binding material to react with the hybrid enzyme of the present invention, and assaying modulations in the enzyme activity. The use of the hybrid enzyme of the present invention and the material having the binding ability allows the presence or amount of the material containing the peptide to be detected, by utilizing that the amount of the material having the binding ability which is bound to the hybrid enzyme is modulated by competition of the peptide introduced into the hybrid enzyme by insertion or substitution and the substance containing the peptide to the material having the binding ability. This process comprises the steps of (1) bringing a sample containing a material to be analyzed, the hybrid enzyme of the present invention and the substance having the binding ability to the foreign peptide introduced into the hybrid enzyme of the present invention by insertion or substitution in contact with one another to form a reaction mixture, (2) bringing the reaction mixture into contact with a substrate to the enzyme(hybrid enzyme), and (3) monitoring changes in the enzyme activity of the hybrid enzyme according to the amount of the material to be analyzed existing in the reaction mixture. Step (2) can also be carried out after the reaction mixture is allowed to reach a steady state or an equilibrium state, and step (1) can be carried out sequentially or concurrently. In step (1), a sample containing the material having the binding ability to the peptide can be reacted with the hybrid enzyme, thereby detecting the presence and amount of the material having the binding ability to the peptide.

As described above, the use of the hybrid enzyme-containing reagent of the present invention allows the presence or amount of the anti-CRP antibody to be directly assayed. Further, the use of a reagent containing the anti-CRP antibody together with the hybrid enzyme of the present invention makes it possible to conduct the assay for indirectly detecting the presence or amount of CRP as an antigen, by binding competition of the hybrid enzyme and CRP to the anti-CRP antibody as a binding material.

The enzyme activity of the hybrid enzyme may be assayed (measured) in accordance with the method for assaying the activity of the original enzyme.

The G6PDHs used for preparing G6PDH-containing hybrid enzymes for assaying a macromolecule material in a homogeneous system, which can maintain the same enzyme activity also after insertion of the foreign peptide and be modulated in the enzyme activity when the material having binding ability to the foreign peptide moiety is bound to the foreign peptide moiety, include one having the amino acid sequence represented by SEQ ID NO: 6 or a sequence obtained by deletion, substitution or addition of one or more amino acids of the amino acid sequence, and one having G6PDH activity even though it is different origin. Preferred examples of the positions at which the foreign peptides are inserted include the positions described above.

The foreign peptides include all the peptides described above. Of these, the peptides as described above are preferably used as CRP.

The hybrid enzyme prepared by using an enzyme other than the G6PDH, into which the foreign peptide containing as a part thereof a CRP-derived peptide is introduced by insertion or substitution, is also prepared in the same manner as described above. The position at which the foreign peptide is introduced by insertion or substitution may also be appropriately selected in the same manner. When the β-galactosidase is used as the enzyme, the position may also be selected, based on the descriptions of FEBS Letters, 434, 23-27 (1998) and FEBS Letters, 438, 267-271 (1998). When the alkaline phosphatase is used, the position may also be selected, based on a method described in Proc. Natl. Acad. Sci. U.S.A., 92 (1995). The use of the thus obtained hybrid enzyme into which the foreign peptide is introduced allows the presence or the amount of the material having the binding ability to the foreign peptide to be directly assayed, and the use of the hybrid enzyme and the material having the binding ability to the foreign peptide such as an antibody in combination makes it possible to conduct the assay for indirectly detecting the presence or the amount of the macromolecule material containing the foreign peptide. These assays may be conducted, based on the assaying operations of CRP or the anti-CRP antibody described above. Further, the use of the hybrid enzyme allows the macromolecule material to be assayed with high sensitivity in the homogeneous system, and this method can also be applied to typical automatic analyzers.

Examples are shown below for illustrating the invention in more detail, but the invention is not construed as being limited by descriptions given therein.

EXAMPLE 1

Construction Plasmid containing G6PDH gene

Five milliliters of LACTOBACILLI MRS BROTH (DIFICO) was inoculated with Leuconostoc mesenteroides, and shake cultured at 26°C for 16 hours to obtain a culture medium. Then, the culture medium was centrifuged at 4°C at 6000 rpm for 10 minutes, and harvested to obtain cells. The cells were suspended in a 10 mM tris (hydroxymethyl) aminomethane (Tris/HCl) buffer containing 1 mM ethylenediaminetetraacetic acid (EDTA) (hereinafter referred to as TE for brevity), and achromopeptitase was added so as to give a final concentration of 300 u/ml, followed by standing at 37°C for 2 hours. Then, SDS and Proteinase K were added so as to give final concentrations of 0.5% and 100 μg/ml, respectively, and the resulting suspension was further allowed to stand at 37°C for 2 hours to conduct bacteriolysis. Genomic DNA of Leuconostoc mesenteroides was extracted as a donor of a G6PDH gene according to a conventional method described in Molecular Cloning (J. Sambrook et al., 2nd Edition, Cold Spring Harbor Laboratory). Operations were hereinafter conducted according to well-known techniques generally employed, which were described in Molecular Cloning (J. Sambrook et al., 2nd Edition, Cold Spring Harbor Laboratory) and the like, unless otherwise specified. Then, for obtaining the G6PDH gene, the polymerase chain reaction (hereinafter referred to as PCR for brevity) was conducted according to the following procedure. The above-mentioned genomic DNA (10 ng) was added as a template DNA, and 0. 1 nmol of each oligonucleotide (primer) represented by SEQ ID NO: 7 and SEQ ID NO: 8, respectively, containing the N-terminal and C-terminal sequences of the G6PDH gene, respectively, was added. Then, the reaction cycle at 94°C for 30 seconds, at 65°C for 30 seconds and at 72°C for 4 minutes was repeated 25 times using a DNA thermal cycler (Perkin Elmer) to amplify DNA fragment. As a result, an about 1. 5-kbp DNA fragment containing the G6PDH gene was specifically amplified. The resulting DNA fragment was ligated to an Eco RV site of cloning vector pBluescript II KS+(Stratagene) to construct plasmid pBSWG.

Then, cloning vector pUC18 was digested with Eco RI and Sal I, and then, an end thereof was made flush. The resulting DNA fragment was ligated to the end made flush to construct plasmid pUCG which could express the G6PDH gene. Further, the 750th from the N-terminal of the G6PDH gene, cytosine, was varied to thymine using the oligonucleotide (primer) represented by SEQ ID NO: 9 and Mutan-K (Takara Shuzo Co., Ltd.) according to the Kunkel method, thereby constructing plasmid pBSMG containing the G6PDH gene having no restriction enzyme Nco I recognition sequence at a position other than the N-terminal, without changing the amino acid sequence. This plasmid was digested with restriction enzymes Nco I and Pst I, and an about 1.5-kbp G6PDH gene was recovered. This gene was ligated to an about 2.7-kbp DNA fragment obtained by digesting plasmid pUCG with restriction enzymes Nco I and Pst I to construct plasmid pUCMG.

EXAMPLE 2

Construction of Recombinant DNA Coding for Fused (Hybrid) Enzyme Having Human CRP-Derived Peptide Ligated between Pro308/Ala309 of G6PDH

Using plasmid pUCMG of Example 1 as a template, and using the oligonucleotide (primer) represented by SEQ ID NO: 7, and the oligonucleotide (primer) represented by SEQ ID NO: 10, which carry the recognition sequence for a restriction enzyme BamH I (BamH I site) at the 5'-terminal of an anti-sense strand sequence upstream from Pro308, the PCR was conducted to obtain an about 0.9-kbp DNA fragment containing a part from the N-terminal to Pro308 of the G6PDH gene, which carry the BamH I site at a downstream site. Similarly, using the oligonucleotide (primer) represented by SEQ ID NO: 11 which carry the BamH I site added at the 5'-terminal of a sense strand sequence downstream from Ala309, and the oligonucleotide (primer) represented by SEQ ID NO: 12 containing the C-terminal anti-sense strand sequence of the G6PDH gene, an about 0.6-kbp DNA fragment containing a part from Ala309 to the C-terminal of the G6PDH gene, which carry the BamH I at an upstream site was obtained.

The fragment of the N-terminal side was digested with restriction enzymes BamH I and Nco I, and the fragment of the C-terminal side was digested with restriction enzymes BamH I and Pst I, followed by ligation with an about 2. 7-kbp DNA fragment obtained by digesting plasmid pUCMG with restriction enzyme Nco I and Pst I. Thus, recombinant pUCMG308B having a BamH I site sequence only at Pro308/Ala309 of the G6PDH gene was constructed. This recombinant was cleaved with restriction enzyme BamH I, and synthetic polynucleotides (a combination of SEQ ID NO: 13 and SEQ ID NO: 14, complemental with each other) having DNA coding for the amino acid sequence represented by SEQ ID NO: 2 were ligated thereto to construct pUCMG308C1. Synthetic nucleotides (a combination of SEQ ID NO: 15 and SEQ ID NO: 16, and a combination of SEQ ID NO: 21 and SEQ ID NO: 22, complemental with each other) having DNA coding for a part (portion) of the amino acid sequence represented by SEQ ID NO: 3 were each ligated to construct pUCMG308C2 and pUCMG308C13. Further, synthetic polynucleotides (a combination of SEQ ID NO: 17 and SEQ ID NO: 18, complemental with each other) having DNA coding for the amino acid sequence represented by SEQ ID NO: 4 were ligated to construct pUCMG308C3, and synthetic polynucleotides (a combination of SEQ ID NO: 19 and SEQ ID NO: 20, complemental with each other) having DNA coding for the amino acid sequence represented by SEQ ID NO: 5 were ligated to construct pUCMG308C5. The respective synthetic nucleotides have the following complemental structures with each other:

5'-gatccgacatgtcgaggaaggcttttgtgtttcccaaagagtcggatacttccg-3' SEQ ID NO: 13

3'- gatccggaagtatccgactctttgggaaacacaaaagccttcctcgacatgtcg-5' SEQ ID NO: 14

5'-gatccgtgctgtcaccagatgagattaacaccatctatcttggcgggg -3' SEQ ID NO: 15

3'- gatccccgccaagatagatggtgttaatctcatctggtgacagcacg-5' SEQ ID NO: 16

5'-gatccctgaagaagggatacactgtgggggcagaagcaagcg -3' SEQ ID NO: 17

3'- gatccgcttgcttctgcccccacagtgtatcccttcttcagg-5' SEQ ID NO: 18

5'-gatcccgggcactgaagtatgaagtgcaaggcgaag -3' SEQ ID NO: 19

3'- gatccttcgccttgcacttcatacttcagtgcccgg-5' SEQ ID NO: 20

5'-gatcctagtgggagacattggaaatgtgaacatgtgggactttgtgg -3' SEQ ID NO: 21

3'- gatcccacaaagtcccacatgttcacatttccaatgtctcccactag-5' SEQ ID NO: 22

EXAMPLE 3

Expression and Extraction of Fused (Hybrid) Enzymes G308C1, G308C2, G308C3, G308C5 and G308C13

Recombinant DNAs pUCMG308C1, pUCMG308C3, pUCMG308C5, pUCMG308C2 and pUCMG308C13 of Example 2 were transformed into E. Coli XL1-Blue. According to the method of Levy et al. (Protein Science, 1, 329-(1992)), the transformed E. Coli was shake cultured in LB medium (DIFCO) at 37°C for 16 hours to obtain a culture medium. The resulting culture medium was inoculated in LB medium to yield a final concentration of 2%, and shake cultured at 37°C for 5 hours. Then, IPTG (isopropyl-β-D-thiogalactopyranoside) was added to achieve a final concentration of 0.5 mM, thereby inducing expression, and the culture was incubated at 37°C for 16 hours, followed by centrifugation at 4°C at 6000 rpm for 10 minutes to obtain the cells. The cells were suspended in a 10 mM Tris/HCl buffer containing 8% sucrose, 0.1% Triton-X and 50mM EDTA, and lysozyme was added thereto so as to give a final concentration of 33 mg/ml. The resulting product was allowed to stand at 37°C for 30 minutes, and then, insoluble material was removed by centrifugation at 15000 rpm for 20 minutes to obtain a solution of each of fused (hybrid) enzymes G308C1, G308C2, G308C3, G308C5 and G308C13. As a control, a wild type enzyme solution was also similarly obtained from plasmid pUCMG.

EXAMPLE 4

Effect of Anti-CRP Antibody to Enzyme Activity of Fused (Hybrid) Enzymes G308C1, G308C2, G308C3, G308C5 and G308C13

The activity of the fused (hybrid) enzyme solutions obtained in Example 3 was assayed in the absence and presence of an anti-CRP antibody, respectively, to examine modulations in enzyme activity due to the binding of the anti-CRP antibody. To 6 μl of a 400-fold dilution of each fused (hybrid) enzyme solution with a 100 mM Tris/HCl buffer (pH 7.8) containing 1% bovine albumin, 3 mM magnesium chloride and 150 mM sodium chloride, 150 μl of a 100 mM Tris/HCl buffer containing 3 mM magnesium chloride and 150 mM sodium chloride (pH 7.8, buffer A), or of a 100-fold antibody dilution of anti-CPR goat antibody with buffer A was added, followed by reaction at 37°C for 5 minutes. Then, 75 μl of buffer A containing 10 mM glucose-6-phosphate (G6P) and 6 mM nicotinamide adenine dinucleotide (NAD) was added thereto, followed by reaction at 37°C for 5 minutes, and the changes in absorbance at a wavelength of 340 nm for 5 minutes were determined as G6PDH activity. Activity ratio of the enzyme activity in the presence of the anti-CRP antibody to that in the absence of the antibody is shown in Table 2. As a result, it is known that the wild type G6PDH indicates no difference in activity between in the absence of the anti-CRP antibody and in the presence thereof, whereas fused (hybrid) enzymes G308C1, G308C2, G308C3, G308C5 and G308C13 are decreased in the enzyme activity in the presence of the anti-CRP antibody, compared to that in the absence thereof. That is to say, in the fused (hybrid) enzymes in which CRP peptides represented by SEQ ID NO: 2, SEQ ID NO: 3, SEQ ID NO: 4 and SEQ ID NO: 5 or a part thereof are inserted at Pro308/Ala309 of G6PDH, it is found that their enzyme activity is inhibited by the binding of the anti-CRP antibody.

Table 2

| | Activity Ratio |
|---|---|
| Wild type | 101% |
| Fused (hybrid) Enzyme G308C1 | 86% |
| G308C2 | 29% |
| G308C3 | 36% |
| G308C5 | 88% |
| G308C13 | 39% |
| Activity ratio (%) = (G6PDH activity in the presence of antibody)/(G6PDH activity in the absence of antibody) X 100 | |

EXAMPLE 5

Assay of Anti-CRP antibody Using Fused (Hybrid) Enzyme G308C1

Modulations in the activity of fused (hybrid) enzyme G308C1 which are dependent on the amount of the anti-CRP antibody were examined. To 50 μl of each of 100-fold, 1000-fold, 10000-fold and 100000-fold dilutions of the anti-CRP monoclonal antibody with buffer A, or to 50 μl of buffer A, 100μl of a 3300-fold dilution of fused (hybrid) enzyme G308C1 diluted with buffer A was added, followed by reaction at 37°C for 5 minutes. Then, 75 μl of buffer A containing 10 mM G6P and 6 mM NAD was added thereto, followed by reaction at 37°C for 5 minutes, and the changes in absorbance at a wavelength of 340 nm for 5 minutes were determined as G6PDH activity. Results thereof are shown in Fig. 1. As known from the results, a phenomenon is observed that the enzyme activity increases with a decrease in the amount of the anti-CRP antibody. That is to say, it is shown that the anti-CRP antibody can be assayed using the fused (hybrid) enzyme in which CRP peptide represented by SEQ ID NO: 2 is inserted at Pro308/Ala309 of G6PDH.

EXAMPLE 6

Assay of CRP Using Fused (Hybrid) Enzyme G308C1

It was examined whether the hybrid enzyme activity inhibited by the binding of the anti-CRP antibody is reactivated (recovered) with an increase in the CRP concentration. To 6 µl of each of CRP solutions of various concentrations (0, 10, 20 and 40 mg/dl), 100 µl of a 3300-fold dilution of a solution of fused (hybrid) enzyme G308C1 diluted with buffer A was added, followed by reaction at 37°C for 3 minutes. Then, 50 µl of a 10000-fold dilution of the anti-CRP monoclonal antibody diluted with buffer A was added thereto. After further reaction at 37°C for 3 minutes, 75 µl of buffer A containing 10 mM G6P and 6 mM NAD was added thereto, followed by reaction at 37°C for 5 minutes, and the changes in absorbance at a wavelength of 340 nm for 5 minutes were determined as G6PDH activity. As shown in Fig. 2, a phenomenon is observed that the activity is reactivated (recovered) with an increase in CRP concentration. That is to say, it is shown that CRP can be assayed using the fused (hybrid) enzyme in which CRP peptide represented by SEQ ID NO: 2 is inserted at Pro308/Ala309 of G6PDH.

EXAMPLE 7

Assay of CRP Using Fused (Hybrid) Enzymes G308C2, G308C3 and G308C5

The assay of CRP was tried in the same manner as with Example 6. Dilutions of fused (hybrid) enzymes G308C2, G308C3 and G308C5 were 5000-fold, 15000-fold and 2000-fold, respectively, and the dilution ratio of anti-CPR goat antibody was 8-fold. As a result, a phenomenon is observed that the activity is reactivated (recovered) with an increase in CRP concentration, as shown in Figs. 3,4 and 5. That is to say, it is shown that CRP can be assayed using the fused (hybrid) enzymes in which CRP peptides represented by SEQ ID NO: 3, SEQ ID NO: 4 and SEQ ID NO: 5 are each inserted at Pro308/Ala309 of G6PDH.

EXAMPLE 8

Construction of Recombinant DNA Coding for Fused (Hybrid) Enzyme Having CRP-Derived Peptide Inserted at Asp306/Val307 of G6PDH

Using a combinations of the oligonucleotides (primers) represented by SEQ ID NO: 7 and SEQ ID NO: 23 and a combination of the oligonucleotides (primers) represented by SEQ ID NO: 12 and SEQ ID NO: 24, recombinant pUCMG306B having a BamH I site only at Asp306/Val307 of the G6PDH gene was constructed, and the synthetic polynucleotides represented by SEQ ID NO: 13 and SEQ ID NO: 14 were ligated thereto to construct recombinant DNA pUCMG306C1, in the same manner as with Example 2.

EXAMPLE 9

Assay of CRP Using Fused (Hybrid) Enzyme G306C1

A solution of fused (hybrid) enzyme G306C1 was obtained in the same manner as with Example 3. Then, the assay of CRP was tried in the same manner as with Example 6. A 500-fold dilution of the fused (hybrid) enzyme solution was used, and the dilution ratio of anti-CPR monoclonal antibody was 3200-fold. As a result, a phenomenon is observed that the activity is reactivated (recovered) with an increase in CRP concentration, as shown in Fig. 6. That is to say, it is shown that CRP can be assayed using the fused (hybrid) enzyme in which CRP peptide represented by SEQ ID NO: 2 is inserted at Asp306/Val307 of G6PDH.

EXAMPLE 10

Construction of Recombinant DNA Coding for Fused (Hybrid) Enzyme Having CRP-Derived Peptide Inserted between Ala309/Asp310 of G6PDH

Using a combinations of the oligonucleotides (primers) represented by SEQ ID NO: 7 and SEQ ID NO: 25 and a combination of the oligonucleotides (primers) represented by SEQ ID NO: 12 and SEQ ID NO: 26, recombinant pUCMG309B having a BamH I site only at Ala309/Asp310 of the G6PDH gene was constructed, and the synthetic polynucleotides represented by SEQ ID NO: 13 and SEQ ID NO: 14 were ligated thereto to construct recombinant DNA pUCMG309C1, in the same manner as with Example 2.

EXAMPLE 11

Assay of CRP Using Fused (Hybrid) Enzyme G309C1

A solution of fused (hybrid) enzyme G309C1 was obtained in the same manner as with Example 3. Then, the assay of CRP was tried in the same manner as with Example 6. A 2500-fold dilution of the fused (hybrid) enzyme solution and a 1000-fold dillution of anti-CRP monoclonal antibody were used, and the dilution ratio of anti-CPR monoclonal antibody was 10000-fold. As a result, a phenomenon is observed that the activity is reactivated (recovered) with an increase in CRP concentration, as shown in Fig. 7. That is to say, it is shown that CRP can be assayed using the fused (hybrid) enzyme in which CRP peptide represented by SEQ ID NO: 2 is inserted at Ala309/Asp310 of G6PDH.

EXAMPLE 12

Construction of Recombinant DNA Coding for Fused (Hybrid) Enzyme Having CRP-Derived Peptide Added to C-Terminal of G6PDH

Using plasmid pBSMG, the oligonucleotide (primer) represented by SEQ ID NO: 27 and Mutan-K (Takara Shuzo Co., Ltd.), plasmid pBSMGCB having a restriction enzyme BamH I sequence added to the C-terminal of the G6PDH gene was constructed according to the Kunkel method. Then, recombinant pUCMGCB having the BamH I site only at the C-terminal was constructed, and the synthetic oligonucleotide represented by SEQ ID NO: 13 was ligated thereto to construct recombinant DNA pUCMGCC1, in the same manner as with Examples 1 and 2.

EXAMPLE 13

Effect of Anti-CRP Antibody to Enzyme Activity of Fused (Hybrid) Enzyme GCC1

A solution of fused (hybrid) enzyme GCC1 was obtained in the same manner as with Example 3. Then, the effect at the time when the anti-CRP antibody was bound to fused (hybrid) enzyme GCC1 was examined by the same procedure as with Example 4. Activity ratio of the enzyme activity in the presence of the anti-CRP antibody to that in the absence of the antibody is shown in Table 3. As a result, it is known that the wild type G6PDH indicates no difference in activity between in the absence of the anti-CRP antibody and in the presence thereof, whereas fused (hybrid) enzyme GCC1 is decreased in the enzyme activity in the presence of the anti-CRP antibody, compared to that in the absence thereof. That is to say, in the fused (hybrid) enzyme in which CRP peptide represented by SEQ ID NO: 2 is added to the C-terminal of G6PDH, it is found that its enzyme activity is inhibited by the binding of the anti-CRP antibody.

Table 3

| | Activity Ratio |
|---|---|
| Wild type | 101% |
| Fused (Hybrid) Enzyme GCC1 | 95% |
| Activity ratio (%) = (G6PDH activity in the presence of antibody)/(G6PDH activity in the absence of antibody) X 100 | |

EXAMPLE 14

Construction of Recombinant DNA Coding for Fused (Hybrid) Enzyme Having CRP-Derived Peptide Inserted between Glu362/Gln363 of G6PDH

Using a combinations of the oligonucleotides (primers) represented by SEQ ID NO: 7 and SEQ ID NO: 28 and a combination of the oligonucleotides (primers) represented by SEQ ID NO: 12 and SEQ ID NO: 29, recombinant pUCMG362B having a BamH I site only at Glu362/Gln363 of the G6PDH gene was constructed, and the synthetic polynucleotides represented by SEQ ID NO: 13 and SEQ ID NO: 14 were ligated thereto to construct recombinant DNA pUCMG362C1, in the same manner as with Example 2.

EXAMPLE 15

Assay of Anti-CRP Antibody Using Fused (Hybrid) Enzyme G362C1

A solution of fused (hybrid) enzyme G362C1 was obtained in the same manner as with Example 3. Then, modulations in the activity of fused (hybrid) enzyme G362C1 with the amount of the anti-CRP antibody were examined in the same manner as with Example 5. As a result, a phenomenon is observed that the activity is increased with an increase in the anti-CRP antibody concentration, as shown in Fig. 8. That is to say, it is shown that an anti-CRP antibody can be assayed using the fused (hybrid) enzyme in which CRP peptide represented by SEQ ID NO: 2 is inserted at Glu362/Gln363 of G6PDH.

EXAMPLE 16

Fused (Hybrid) Enzyme Having a Part of CRP-Derived Peptide Represented by SEQ ID NO: 2 Inserted into G6PDH

Synthetic nucleotides (a combination of SEQ ID NO: 32 and SEQ ID NO: 33, and a combination of SEQ ID NO: 34 and SEQ ID NO: 35) having DNA sequence coding for a part of the amino acid sequence represented by SEQ ID NO: 2 were each ligated to recombinant pUCMG306B having a BamH I site only at Asp306/Va1307 of the G6PDH gene constructed in Example 8 to construct recombinant DNAs pUCMG308C15 and pUCMG308C18 respectively. Similarly, using recombinant pUCMG308B in Example 2, recombinant DNAs pUCMG308C15 and pUCMG308C18 were each constructed. Using these, fused (hybrid) enzymes G306C15, G306C18, G308C15 and G308C18 were obtained by the same procedure as with Example 3. As to these fused (hybrid) enzymes and fused (hybrid) enzymes G306C1 and G308C1 obtained in Example 9 and Example 3, the effect at the time when the anti-CRP antibody was bound to each fused (hybrid) enzyme was examined by the same procedure as with Example 4. The enzyme activity in the presence of the anti-CRP antibody (a 1000-fold dilution of the anti-CRP monoclonal antibody) to that in the absence of the antibody is shown in Table 4 as the activity ratio. Then, using fused (hybrid) enzymes G306C1, G306C15 and G306C18, a comparison of CRP assaying sensitivity was made. To 10µl of each of CRP solutions having various concentrations (0, 5, 10, 20 and 40 mg/dl), 250 µl of a mixed solution of each fused (hybrid) enzyme and the anti-CRP monoclonal antibody diluted with buffer A was added, followed by reaction at 37°C for 5 minutes. Then, 125 µl of buffer A containing 10 mM G6P and 6 mM NAD was added thereto, followed by further reaction at 37°C for 5 minutes, and the changes in absorbance at a wavelength of 340 nm for 5 minutes were determined as G6PDH activity. The dilution ratios of fused (hybrid) enzymes G306C1, G306C15 and G306C18 were 1040-fold, 6240-fold and 58500-fold, respectively, and all the dilution ratio of anti-CPR monoclonal antibody was 15600-fold. Results thereof are shown in Fig. 9. The results show the possibility of controlling the assaying sensitivity by the selection of the enzyme site into which the peptide is inserted, and/or by the selection of the peptide length to be inserted.

Table 4

| | Activity Ratio |
|---|---|
| Wild type | 100.4% |
| Fused (Hybrid) Enzyme G306C1 | 53.1% |
| G306C15 | 54.0% |
| G306C18 | 16.2% |
| G308C1 | 78.9% |
| G308C15 | 89.8% |
| G308C18 | 94.6% |

CRP peptide sequences inserted into the respective fused (hybrid) enzymes are shown below:

G306C1, G308C1;

Asp Met Ser Arg Lys Ala Phe Val Phe Pro Lys Glu Ser Asp Thr Ser

G306C15, G308C15;

Asp Met Ser Arg Lys Ala Phe Val Phe Pro Lys Glu Ser

G306C18, G308C18;

Arg Lys Ala Phe Val Phe Pro Lys Glu Ser

EXAMPLE 17

Fused (Hybrid) Enzymes Having CRP-Derived Peptides Inserted into N-Terminal, at Asp294/Ser295 and at Leu302/Glu303 of G6PDH

Using plasmid pBSMG, the oligonucleotide (primer) represented by SEQ ID NO: 36 and Mutan-K (Takara Shuzo Co., Ltd.), plasmid pBSMGNB having a BamH I site added to the N-terminal of the G6PDH gene was constructed according to the Kunkel method. Then, recombinant pUCMGNB having the BamH I site only at the N-terminal was constructed by the same procedure as with Examples 1 and 2. Further, using a combination of the oligonucleotides (primers) represented by SEQ ID NO: 7 and SEQ ID NO: 37 and a combination of the oligonucleotides (primers) represented by SEQ ID NO: 12 and SEQ ID NO: 38, recombinant pUCMG294B having a BamH I site only at Asp294/Ser295 of the G6PDH gene was constructed, and using a combination of the oligonucleotides (primers) represented by SEQ ID NO: 7 and SEQ ID NO: 39 and a combination of the oligonucleotides (primers) represented by SEQ ID NO: 12 and SEQ ID NO: 40, recombinant pUCMG302B having a BamH I site only at Leu302/Glu303 of the G6PDH gene was constructed, by the same procedure as with Example 2. Then, the synthetic polynucleotides represented by SEQ ID NO: 34 and SEQ ID NO: 35 were ligated thereto to construct recombinant DNAs pUCMGNC18, pUCMG294C18 and pUCMG302C18. Solutions of fused (hybrid) enzymes GNC18, G294C18 and G302C18 were obtained by the same procedure as with Example 3. The effect at the time when the anti-CRP antibody was bound to each fused (hybrid) enzyme was examined by the same procedure as with Example 4. Activity ratio of the enzyme activity in the presence of the anti-CRP antibody (a 1000-fold dilution of the anti-CRP monoclonal antibody) to that in the absence of the antibody is shown in Table 5. As a result, it is known that the wild type G6PDH indicates no difference in activity between in the absence of the anti-CRP antibody and in the presence thereof, whereas fused (hybrid) enzymes GNC18, G294C18 and G302C18 are decreased in the enzyme activity in the presence of the anti-CRP antibody, compared to that in the absence thereof. That is to say, in all the fused (hybrid) enzymes in which CRP peptides are inserted into the N-terminal, at Asp294/Ser295 and at Leu302/Glu303 of G6PDH, it is found that their enzyme activity is inhibited by the binding of the anti-CRP antibody.

Table 5

| | Activity Ratio |
|---|---|
| Wild type | 100.3% |
| Fused (Hybrid) Enzyme GNC18 | 90.2% |
| G294C18 | 51.4% |
| G302C18 | 57.5% |

EXAMPLE 18

Fused (Hybrid) Enzyme Having CRP-Derived Peptides Inserted into Two Positions, at Asp306/Va1307 and C-Terminal of G6PDH

The synthetic oligonucleotides represented by SEQ ID NO: 32 and SEQ ID NO: 33 were ligated to recombinant pUCMGCB constructed in Example 12 to construct recombinant DNA pUCMGCC15 by the same procedure as with Example 2, and this recombinant DNA was digested with restriction enzymes Bpu1102 I and Pst I to recover an about 0.5-kbp fragment. Further, recombinant DNA pUCMG306C18 constructed in Example 16 was digested with restriction enzymes Nco I and Bpu1102 I to recover an about 1.0-kbp fragment. Still further, recombinant DNA pUCMG constructed in Example 1 was digested with restriction enzymes Nco I and Pst I to recover an about 2.7-kbp fragment. These three recovered fragments were ligated, thereby constructing recombinant DNA pUCMG306C18+CC15 in which synthetic oligonucleotides coding for CRP-derived peptide were inserted into two positions of the G6PDH gene. By the same procedure as with Example 3, a solution of fused (hybrid) enzyme G306C18+CC15 was obtained. The effect at the time when the anti-CRP antibody was bound to the fused (hybrid) enzyme was examined by the same procedure as with Example 4. Activity ratio of the enzyme activity in the presence of the anti-CRP antibody (a 1000-fold dilution of the anti-CRP monoclonal antibody) to that in the absence of the antibody is shown in Table 6. As a result, it is known that the wild type G6PDH indicates no difference in activity between in the absence of the anti-CRP antibody and in the presence thereof, whereas fused (hybrid) enzyme G306C18+CC15 is decreased in the enzyme activity in the presence of the anti-CRP antibody, compared to that in the absence thereof. That is to say, in the fused (hybrid) enzyme in which a part of CRP peptide represented by SEQ ID NO: 2 are inserted into two positions, between Asp306/Val307 and the C-terminal, it is found that its enzyme activity is inhibited by the binding of the anti-CRP antibody.

Table 6

| | Activity Ratio |
|---|---|
| Wild type | 99.9% |
| Fused (Hybrid) Enzyme G306C18+CC15 | 15.2% |

EXAMPLE 19

Fused (Hybrid) Enzyme Having Human CRP-Derived Peptide Substituted for a part from Val307 to Ala309 of G6PDH

The about 0.9-kbp DNA fragment containing from the N-terminal to Asp306 of the G6PDH gene, in which the BamH I site was added to a downstream site, prepared in Example 8, and the about 0.6-kbp DNA fragment containing from Asp310 to the C-terminal of the G6PDH gene, in which the BamH I site was added to an upstream site, prepared in Example 10, were used with each other to construct recombinant pUCMG306d3B in which the BamH I site was substituted for a part from Val307 to Ala309 of the G6PDH gene, by the same procedure as with Example 2. The synthetic oligonucleotides represented by SEQ ID NO: 13 and SEQ ID NO: 14 were ligated thereto to construct recombinant DNA pUCMG306d3C1. A solution of fused (hybrid) enzyme G306d3C1 was obtained by the same procedure as with Example 3. Then, CRP was assayed in the same manner as with Example 6. A 5000-fold dilution of the fused (hybrid) enzyme solution was used, and the dilution ratio of anti-CPR monoclonal antibody was 10000-fold. As a result, a phenomenon is observed that the activity is reactivated (recovered) with an increase in CRP concentration, as shown in Fig. 10. That is to say, it is shown that CRP can be assayed using the fused (hybrid) enzyme in which CRP peptide represented by SEQ ID NO: 2 is substituted for from Val307 to Ala309 of G6PDH.

Example 20

Fused (Hybrid) Enzyme Having Tyr Substituted for Asp306 of G6PDH and CRP-Derived Peptide Ligated at Tyr306/Val307 Without Addition of Recognition Sequence for Restriction Enzyme

Using a combinations of the oligonucleotides (primers) represented by SEQ ID NO: 7 and SEQ ID NO: 41 and a combination of the oligonucleotides (primers) represented by SEQ ID NO: 12 and SEQ ID NO: 42, recombinant pUCMG306E having the recognition sequence for a restriction enzyme by substituting Tyr for Asp306 of the G6PDH gene was constructed, and the synthetic oligonucleotides represented by SEQ ID NO: 43 and SEQ ID NO: 44 were ligated thereto to construct recombinant DNA pUCMG306EC18, by the same procedure as with Example 2. By the same procedure as with Example 3, a solution of fused (hybrid) enzyme G306EC18 was obtained. Then, the effect at the time when the anti-CRP antibody was bound to the fused (hybrid) enzyme was examined by the same procedure as with Example 4. Activity ratio of the enzyme activity in the presence of the anti-CRP antibody (a 1000-fold dilution of the anti-CRP monoclonal antibody) to that in the absence of the antibody is shown in Table 7. As a result, it is known that the wild type G6PDH indicates no difference in activity between in the absence of the anti-CRP antibody and in the presence thereof, whereas fused (hybrid) enzyme G306EC18 is decreased in the enzyme activity in the presence of the anti-CRP antibody, compared to that in the absence thereof. That is to say, in the fused (hybrid) enzyme in which Tyr is substituted for Asp306 of G6PDH and a part of CRP peptide represented by SEQ ID NO: 2 is inserted at Tyr306/Val307, it is found that its enzyme activity is inhibited by the binding of the anti-CRP antibody.

Table 7

| | Activity Ratio |
|---|---|
| Wild type | 101.0% |

Table 7 (continued)

| | Activity Ratio |
|---|---|
| Fused (Hybrid) Enzyme G306EC18 | 85.0% |

EXAMPLE 21

Fused (Hybrid) Enzyme Having Hepatitis B Virus preS2 Antigen-Derived Peptide Inserted at Asp306/Va1307 of G6PDH

The synthetic oligonucleotides represented by SEQ ID NO: 47 and SEQ ID NO: 48, which code for a partial sequence (SEQ ID NO: 46) of preS2 antigen represented by SEQ ID NO: 45 (S. Usuda, et al., J. Virol. Methods, 80, 97-112 (1999)), were ligated to pUCMG306B prepared in Example 8 to construct recombinant DNA pUCMG306H1. A solution of fused (hybrid) enzyme G306H1 was obtained in the same manner as with Example 3. Then, modulations in the activity of fused (hybrid) enzyme G306H1 with the amount of the anti-preS2 antibody were examined in the same manner as with Example 5. As a result, a phenomenon is observed that the enzyme activity decreases with an increase in the amount of the anti-preS2 antibody, as shown in Fig. 11. That is to say, it is shown that the anti-preS2 antibody can be assayed using the fused (hybrid) enzyme in which the preS2 peptide represented by SEQ ID NO: 46 is inserted at Asp306/Va1307 of G6PDH.

EXAMPLE 22

Fused (Hybrid) Enzyme Having Parathyroid Hormone (PTH)-Derived Peptide Inserted at Asp306/Va1307 of G6PDH

The synthetic oligonucleotides represented by SEQ ID NO: 51 and SEQ ID NO: 52, which code for a partial sequence (SEQ ID NO: 50) of PTH represented by SEQ ID NO: 49 (J. H. Habener et al., Metabolic Bone Disease, 2nd Edition, 69, WB Saunders, Philadelphia (1999)), were ligated to pUCMG306B prepared in Example 8 to construct recombinant DNA pUCMG306P1. A solution of fused (hybrid) enzyme G306P1 was obtained in the same manner as with Example 3. Then, PTH was assayed in the same manner as with Example 6. A 2000-fold dilution of the fused (hybrid) enzyme solution was used, and the dilution ratio of anti-PTH monoclonal antibody was 8000-fold. As a result, a phenomenon is observed that the activity is reactivated (recovered) with an increase in the PTH concentration, as shown in Fig. 12. That is to say, it is shown that PTH can be assayed using the fused (hybrid) enzyme in which the PTH peptide represented by SEQ ID NO: 50 is inserted at Asp306/Va1307 of G6PDH.

EXAMPLE 23

Construction of Plasmid Containing β-Galactosidase Gene

E. coli ATCC 25922 was inoculated into 3 ml of LB media (DIFCO), and shake cultured at 37°C for 16 hours to obtain a culture medium. Then, the culture medium was centrifuged at 4°C at 6000 rpm for 10 minutes, and harvested to obtain cells. Genomic DNA of E. coli was extracted from the resulting cells according to a conventional method described in Molecular Cloning (J. Sambrook et al., 2nd Edition, Cold Spring Harbor Laboratory). Then, for obtaining the β-galactosidase gene, the PCR was conducted according to the following procedure. That is to say, 10 ng of the above-mentioned genomic DNA as a template DNA and 0.1 nmol of each of the oligonucleotides (primers) represented by SEQ ID NO: 53 and SEQ ID NO: 54 which contain the N-terminal and C-terminal sequences of the β-galactosidase gene respectively, which is described in A. Kalnins et al., EMBO J., 2, 593- (1983), were added. Then, the reaction cycle at 94°C for 30 seconds, at 60°C for 1 minute and at 72°C for 5 minutes was repeated 25 times using a DNA thermal cycler (Perkin Elmer) to amplify DNA fragment. As a result, an about 3.1-kbp DNA fragment containing the β-galactosidase gene was specifically amplified. On the other hand, cloning vector pUC19 was digested with Eco RI and Hind III, and an end thereof was made flush with a Klenow fragment. The DNA fragment obtained by the PCR was ligated thereto to construct plasmid pUCB which was cloned in the state that a gene coding for β-galactosidase could be expressed.

EXAMPLE 24

Construction of Recombinant DNA Coding for Fused (Hybrid) Enzyme Having CRP-Derived Peptide Inserted at Va1796/Ser797 of β-galactosidase

Using plasmid pUCB obtained in Example 23 as a template, and using the oligonucleotides (primers) represented by SEQ ID NO: 53 and SEQ ID NO: 55 and the oligonucleotides (primers) represented by SEQ ID NO: 54 and SEQ ID NO: 56, the PCR was conducted in the same manner as with Example 2 to obtain an about 2. 4-kbp DNA fragment and an about 0. 7-kbp DNA fragment in which BamH I sites were added at a site downstream from Val796 and at a site upstream from Ser797, respectively. The fragment of the N-terminal side was digested with restriction enzymes Sac I and BamH I to obtain an about 0.4-kbp DNA fragment, and the fragment of the C-terminal side was digested with restriction enzymes BamH I and Nde I to obtain an about 0. 6-kbp DNA fragment. These fragments were ligated to an about 4.8-kbp DNA fragment obtained by digesting plasmid pUCB with restriction enzymes Sac I and Nde I to construct recombinant pUCB796B having a BamH I site only at Va1796/Ser797 of the β-galactosidase gene. The synthetic polynucleotides represented by SEQ ID NO: 13 and SEQ ID NO: 14 were ligated thereto to construct pUCB796Cl.

EXAMPLE 25

Effect of Anti-CRP Antibody to Enzyme Activity of Fused (Hybrid) Enzyme B796C1

A solution of fused (hybrid) enzyme B796C1 was obtained by the same procedure as with Example 3 with the exception that pUCB796C1 was used as the recombinant. Then, the β-galactosidase activity of the fused (hybrid) enzyme solution was assayed in the absence and presence of the anti-CRP antibody, respectively, according to the method of Villaverde et al. (FEBS Letters, 434, 23- (1998)), to examine modulations in enzyme activity due to the binding of the anti-CRP antibody. To 6 µl of a 50-fold dilution of the fused (hybrid) enzyme solution with a 100 mM phosphate buffer containing 0.1 M 2-mercaptoethanol and 1.0 mM magnesium chloride (pH 7.3, hereinafter referred to as buffer B for brevity), 150 µl of buffer B or a 7500-fold dilution of the anti-CRP monoclonal antibody diluted with buffer B was added, followed by reaction at 37°C for 5 minutes. Then, 24 µl of buffer B containing 17 µM o-nitrophenyl-β-D-galactopyranocide (hereinafter referred to as solution ONPG for brevity) was added thereto, followed by reaction at 37°C for 5 minutes, and the changes in absorbance at a wavelength of 405 nm for 5 minutes after adding solution ONPG were determined as β-galactosidase activity. Activity ratio of the enzyme activity in the presence of the anti-CRP antibody to that in the absence thereof was taken. As a result, it is known that the wild type β-galactosidase indicates no difference in activity between in the absence of the anti-CRP antibody and in the presence thereof, whereas fused (hybrid) enzyme B796C1 is increased in the enzyme activity in the presence of the anti-CRP antibody, compared to that in the absence thereof, as shown in Table 8. That is to say, in the fused (hybrid) enzyme in which CRP peptide represented by SEQ ID NO: 2 is inserted at Va1796/Ser797 of β-galactosidase, it is found that its enzyme activity is amplified by the binding of the anti-CRP antibody.

Table 8

| | Activity Ratio |
|---|---|
| Wild type | 100.2% |
| Fused (Hybrid) Enzyme B796C1 | 170.5% |

EXAMPLE 26

Assay of Anti-CRP Antibody Using Fused (Hybrid) Enzyme B796C1

Modulations in the activity of fused (hybrid) enzyme B796C1 which are dependent on the amount of the anti-CRP antibody were examined. To 50 µl of each of 1000-fold, 10000-fold and 100000-fold dilutions of the anti-CRP monoclonal antibody with buffer B, or to 50 µl of buffer B, 100 µl of a 1250-fold dilution of fused (hybrid) enzyme B796C1 diluted with buffer B was added, followed by reaction at 37°C for 5 minutes. Then, 24 µl of solution ONPG was added thereto, followed by reaction at 37°C for 5 minutes. Then, the changes in absorbance at a wavelength of 405 nm for 5 minutes after adding solution ONPG were determined as β-galactosidase activity. As a result, a phenomenon is observed that the enzyme activity increases with a increase in the amount of the anti-CRP antibody, as shown in Fig. 13. That is to say, it is shown that the anti-CRP antibody can be assayed using the fused (hybrid) enzyme in which CRP peptide represented by SEQ ID NO: 2 is inserted at Va1796/Ser797 of β-galactosidase.

EXAMPLE 27

Assay of CRP Using Fused (Hybrid) Enzyme B796C1

Using fused (hybrid) enzyme B796C1 and the anti-CRP antibody, CRP was assayed. To 6 μl of each of CRP solutions having various concentrations (0, 10, 20 and 40 mg/dl), 100 μl of a 1250-fold dilution of a solution of fused (hybrid) enzyme B796C1 diluted with buffer B was added, followed by reaction at 37°C for 3 minutes. Then, 50 μl of a 7500-fold dilution of the anti-CRP monoclonal antibody diluted with buffer B was added thereto. After further reaction at 37°C for 3 minutes, 24 μl of solution ONPG was added thereto, followed by reaction at 37°C for 5 minutes, and the changes in absorbance at a wavelength of 405 nm for 5 minutes after adding solution ONPG were determined as β-galactosidase activity. As a result, a phenomenon is observed that the enzyme activity increased by the binding of the anti-CRP antibody is restored (recovered) with an increase in CRP concentration, as shown in Fig. 14. That is to say, it is shown that CRP can be assayed using the fused (hybrid) enzyme in which CRP peptide represented by SEQ ID NO: 2 is inserted at Va1796/Ser797 of β-galactosidase.

EFFECT OF INVENTION

Using the hybrid enzyme of the present invention into which the foreign peptide is inserted, the presence and the amount of the material having the binding ability to the foreign peptide can be directly assayed. Further, when the hybrid enzyme and the material having the binding ability to the foreign peptide are used in combination, the presence and the amount of a macromolecule material containing the foreign peptide can be indirectly detected and assayed. Furthermore, the use of the hybrid enzyme of the present invention makes it possible to assay a trace amount of CRP in a sample by a homogeneous colorimetry, which enables early diagnosis of inflammation in the tissue, and further early diagnosis of diseases. Moreover, various macromolecule materials can be easily assayed in homogeneous systems.

## Claims

**1.** A hybrid enzyme which has a partial substitution or an insertion of a peptide containing a part of an amino acid sequence represented by SEQ ID NO:1, in which

said hybrid enzyme has the same enzyme activity as an original enzyme without the substitution or the insertion of said peptide,
and said hybrid enzyme activity is modulated when a material having binding ability to said peptide introduced by the substitution or the insertion is bound to the peptide moiety.

**2.** The hybrid enzyme according to claim 1, in which the peptide comprises an amino acid sequence having at least 6 or more sequential amino acid residues selected from the amino acid sequence of SEQ ID NO: 1.

**3.** The hybrid enzyme according to claim 2, in which the peptide has a property of being capable of binding to a material having binding ability to C-reactive protein.

**4.** The hybrid enzyme according to claim 1, in which the peptide comprises an amino acid sequence having at least 6 or more sequential amino acid residues selected from any one of SEQ ID NO: 2 through SEQ ID NO: 5.

**5.** The hybrid enzyme according to claim 1, in which the original enzyme is a glucose-6-phosphate dehydrogenase, a β-galactosidase or an alkaline phosphatase.

**6.** The hybrid enzyme according to claim 1, in which the material having binding ability to the peptide is an antibody.

**7.** A reagent for measurement of C-reactive protein comprising the hybrid enzyme according to any one of claims 1 through 6.

**8.** The reagent according to claim 7 further comprising an anti-C-reactive protein antibody.

**9.** A kit for measurement of C-reactive protein containing a reagent comprising the hybrid enzyme according to any one of claims 1 through 6.

**10.** The kit according to claim 9 further comprising an anti-C-reactive protein antibody.

**11.** A method for measurement of C-reactive protein which is **characterized in** using the hybrid enzyme according to any one of claims 1 through 6.

**12.** The method according to claim 11 further comprising using an anti-C-reactive protein antibody in combination.

**13.** A method for measurement of C-reactive protein comprising bringing a sample containing C-reactive protein, the enzyme according to any one of claims 1 through 6 and an anti-C-reactive protein antibody into contact with one another, then measuring activity of the enzyme, and determining the amount of C-reactive protein in the sample based on the resulting enzyme activity.

**14.** A hybrid enzyme having a peptide introduced into a specific position of a glucose-6-phosphate dehydrogenase by insertion or substitution.

**15.** The hybrid enzyme according to claim 14, in which the specific position is a position at which the glucose-6-phosphate dehydrogenase activity can be maintained even by the insertion or substitution of a peptide having 6 or more amino acid residues.

**16.** The hybrid enzyme according to claim 14, in which the specific position is a position at which the glucose-6-phosphate dehydrogenase activity is modulated when a material having binding ability to the peptide introduced by insertion or substitution is bound to said peptide.

**17.** The hybrid enzyme according to claim 14, in which the specific position is any position selected from the group consisting of the position between 294-295, between 302-310, between 362-363, the N-terminal and the C-terminal of the amino acid sequence of glucose-6-phosphate dehydrogenase represented by SEQ ID NO: 6.

**18.** The hybrid enzyme according to claim 14, in which the peptide is selected from the amino acid sequence of C-reactive protein.

**19.** The hybrid enzyme according to claim 14, in which the peptide has a character that there is a material having binding ability specifically to the part of the hybrid enzyme in which the peptide is substituted or inserted.

**20.** A reagent for measurement of a material containing the peptide introduced into the hybrid enzyme according to any one of claim 14 through 19 by insertion or substitution, which comprises the hybrid enzyme according to any one of claims 14 through 19.

**21.** A kit for measurement of a material containing the peptide introduced into the hybrid enzyme according to any one of claim 14 through 19 by insertion or substitution, which comprises the hybrid enzyme according to any one of claims 14 through 19.

**22.** A method for measurement of a material containing the peptide introduced into the hybrid enzyme according to any one of claim 14 through 19 by insertion or substitution, which is **characterized in** using the hybrid enzyme according to any one of claims 14 through 19.

**23.** A method for measurement of a material containing the peptide, which comprises using the hybrid enzyme according to any one of claims 14 through 19 in combination with a material having binding ability to the peptide introduced into the hybrid enzyme by insertion or substitution.

**24.** A method for measurement of a material containing said peptide introduced into the hybrid enzyme according to any one of claim 14 through 19, which comprises bringing the hybrid enzyme according to any one of claims 14 through 19, a sample containing a material containing the peptide introduced into said hybrid enzyme by insertion or substitution and a material having binding ability to said peptide into contact with one another, then measuring activity of said hybrid enzyme, and determining the amount of the material containing said peptide in the sample based on the resulting enzyme activity.

**25.** A reagent for measurement of a material having binding ability to the peptide introduced into said hybrid enzyme according to any one of claim 14 through 19 by insertion or substitution, which comprises the hybrid enzyme according to any one of claims 14 through 19.

**26.** A kit for measurement of a material having binding ability to the peptide introduced into said hybrid enzyme according to any one of claim 14 through 19 by insertion or substitution, which comprises the hybrid enzyme according to any one of claims 14 through 19.

**27.** A method for measurement of a material having binding ability to the peptide introduced into said hybrid enzyme according to any one of claim 14 through 19 by insertion or substitution, which comprises using the hybrid enzyme-according to any one of claims 14 through 19.

**28.** A method for measurement of a material having binding ability to said peptide introduced into the hybrid enzyme according to any one of claim 14 through 19, which comprises bringing the hybrid enzyme according to any one of claims 14 through 19 into contact with a sample containing a material having binding ability to the peptide, then measuring an activity of said hybrid enzyme, and determining the amount of the material having binding ability to said peptide in the sample based on the resulting enzyme activity.

**29.** A gene coding for a hybrid enzyme comprising an amino acid sequence into which a foreign peptide is introduced by substitution or insertion at any position selected from the group consisting of the position between 294-295, between 302-310, between 362-363, the N-terminal and the C-terminal of the amino acid sequence of glucose-6-phosphate dehydrogenase represented by SEQ ID NO: 6.

**30.** A recombinant DNA, which is **characterized in** inserting the hybrid enzyme gene according to claim 29 into a vector DNA.

**31.** A transformant or a transductant comprising the recombinant DNA according to claim 30.

**32.** A method for producing a protein having enzyme activity of glucose-6-phosphate dehydrogenase and a property that the glucose-6-phosphate dehydrogenase activity is modulated when a material having binding ability to an amino acid sequence introduced into glucose-6-phosphate dehydrogenase by substitution or insertion is bound to the amino acid sequence, which comprises cultivating the transformant or the transductant according to claim 31, and collecting the protein.

**33.** A gene coding for a hybrid enzyme comprising an amino acid sequence into which an amino acid sequence, which can be cleaved with a restriction enzyme, is introduced by substitution or insertion at any position selected from the group consisting of the Asp294 position, the Leu302 to Asp310 positions, the Glu362 position, the N-terminal and the C-terminal of the amino acid sequence of glucose-6-phosphate dehydrogenase represented by SEQ ID NO: 6.

**34.** A recombinant DNA, which is **characterized in** inserting the hybrid enzyme gene according to claim 33 into a vector DNA.

**35.** A hybrid enzyme in which a peptide selected from an amino acid sequence represented by SEQ ID NO: 1 is introduced into a specific position of a β-galactosidase by insertion or substitution.

**36.** The hybrid enzyme according to claim 35, in which the specific position is either selected from the position between 280-281 and between796-797 of an amino acid sequence of a β-galactosidase represented by SEQ ID NO: 30.

**37.** A gene coding for the hybrid enzyme according to claim 36.

**38.** A recombinant DNA, which is **characterized in** inserting the hybrid enzyme gene according to claim 37 into a vector DNA.

**39.** A transformant or a transductant comprising the recombinant DNA according to claim 38.

**40.** A method for producing a protein having an enzyme activity of a β-galactosidase and a property that the β-galactosidase activity is modulated when a material having binding ability to an amino acid sequence introduced into the β-galactosidase by substitution or insertion is bound to the amino acid sequence, which comprises cultivating the transformant or the transductant according to claim 39, and collecting the protein.

**41.** A hybrid enzyme in which a peptide selected from an amino acid sequence represented by SEQ ID NO: 1 is introduced into a specific position of an alkaline phosphatase by insertion or substitution.

**42.** The hybrid enzyme according to claim 41, in which the specific position is any one selected from the position between 167-168, between 168-169, btween 407-408, between 91-93 and between 169-177 of an amino acid sequence of an alkaline phosphatase represented by SEQ ID NO: 31.

**43.** A gene coding for the hybrid enzyme according to claim 42.

**44.** A recombinant DNA, which is **characterized in** inserting the hybrid enzyme gene according to claim 43 into a vector DNA.

**45.** A transformant or a transductant comprising the recombinant DNA according to claim 44.

**46.** A method for producing a protein having an enzyme activity of an alkaline phosphatase and a property that the alkaline phosphatase activity is modulated when a material having binding ability to an amino acid sequence introduced into the alkaline phosphatase by substitution or insertion is bound to the amino acid sequence, which comprises cultivating the transformant or the transductant according to claim 45, and collecting the protein.

FIG.1

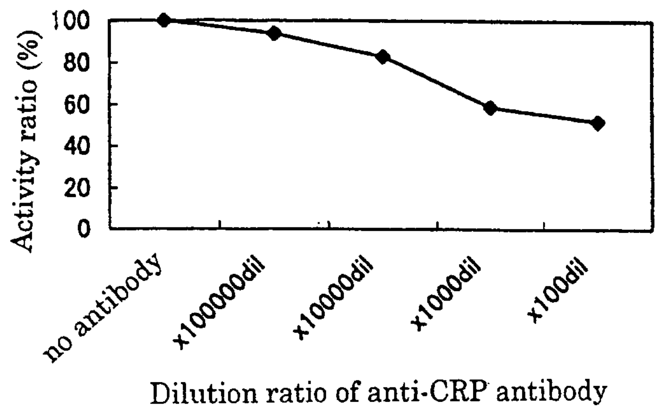


$$\text{Activity ratio (\%)} = \frac{\text{G6PDH activity in the presence of antibody}}{\text{G6PDH activity in the absence of antibody}} \times 100$$

FIG.2

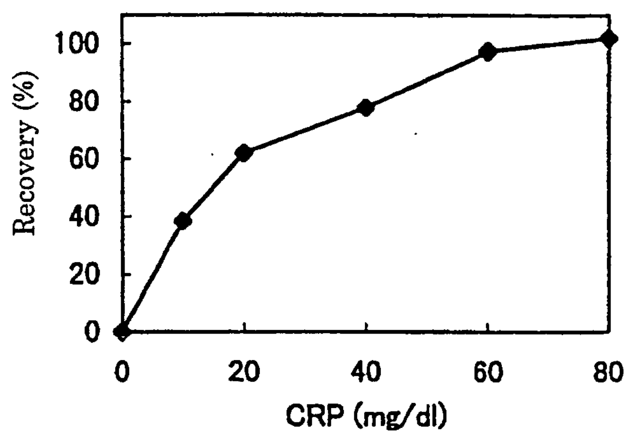

FIG.3

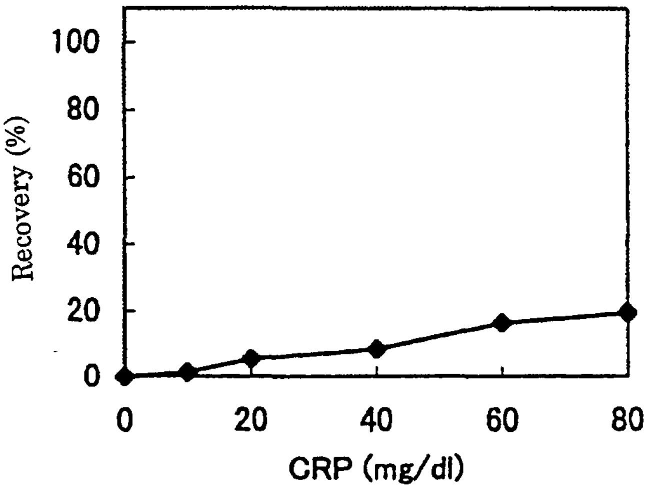
Recovery (%)
100
80
60
40
20
0
0
20
40
60
80
CRP (mg/dl)

FIG.4

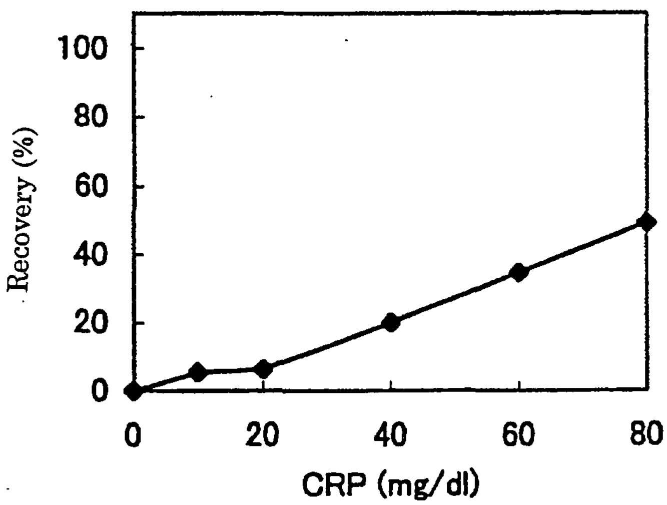
Recovery (%)
100
80
60
40
20
0
0
20
40
60
80
CRP (mg/dl)

FIG.5

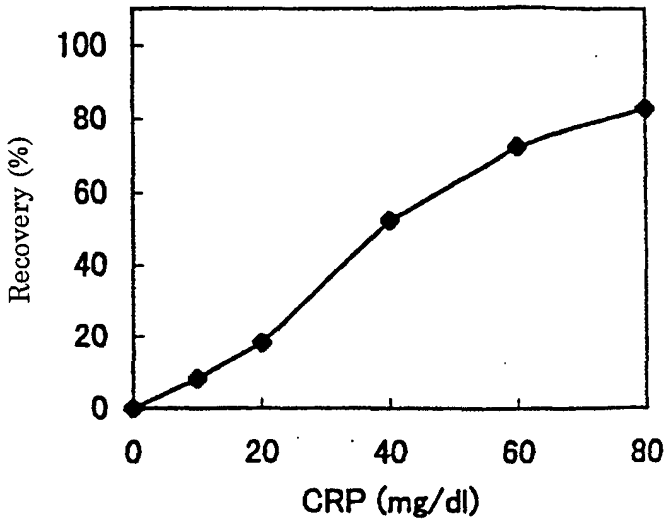
100
80
60
40
20
0
Recovery (%)
0
20
40
60
80
CRP (mg/dl)

FIG.6

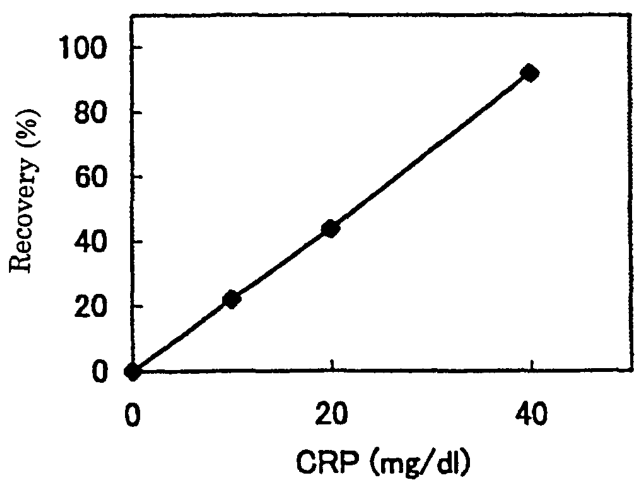
100
80
60
40
20
0
Recovery (%)
0
20
40
CRP (mg/dl)

FIG.7

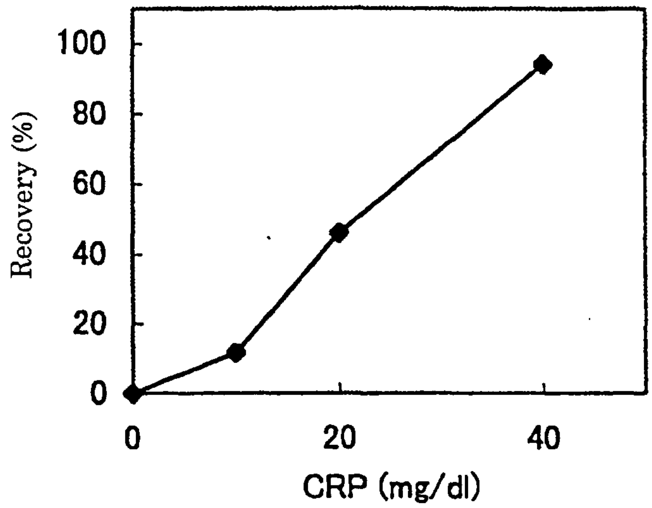
100
80
60
40
20
0
Recovery (%)
0
20
40
CRP (mg/dl)

FIG.8

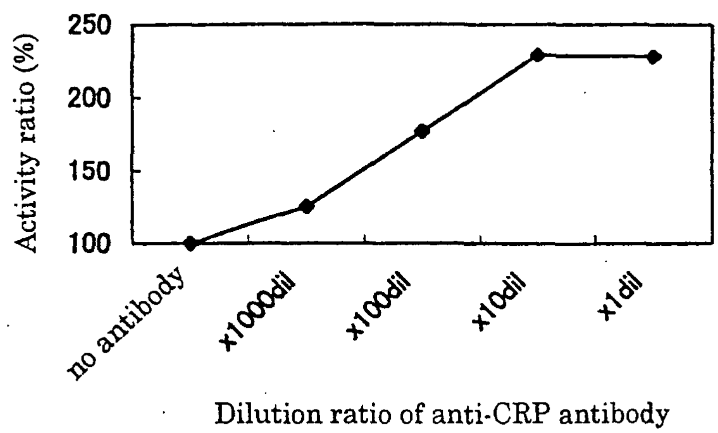
250
200
150
100
Activity ratio (%)
no antibody
x1000dil
x100dil
x10dil
x1dil
Dilution ratio of anti-CRP antibody

FIG.9

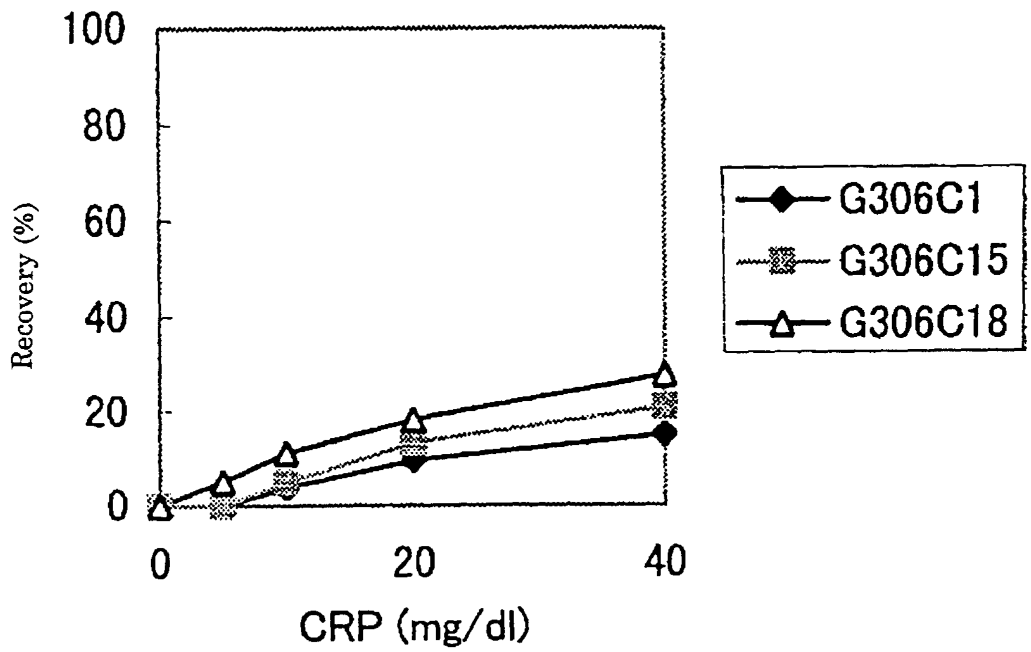
100
80
60
40
20
0
Recovery (%)
0
20
40
CRP (mg/dl)
G306C1
G306C15
G306C18

FIG.10

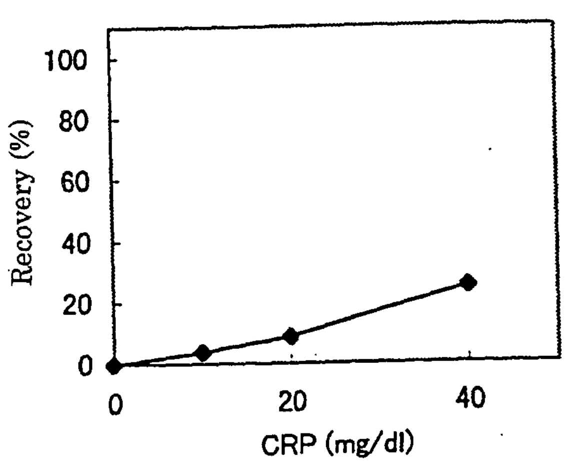
100
80
60
40
20
0
Recovery (%)
0
20
40
CRP (mg/dl)

FIG.11

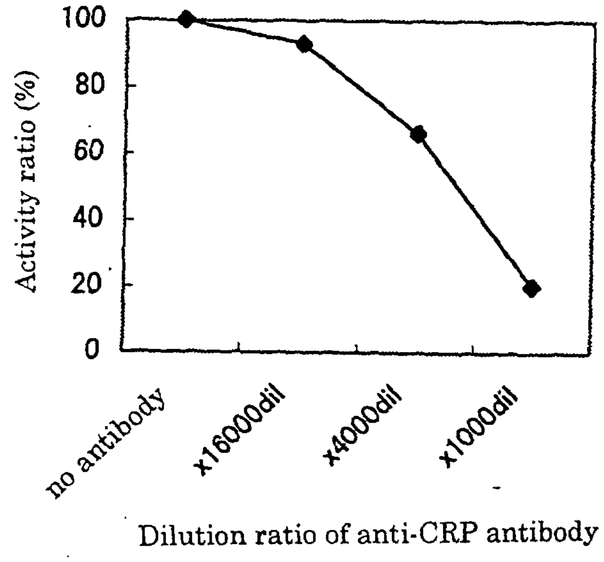
100
80
60
40
20
0
Activity ratio (%)
no antibody
x16000dil
x4000dil
x1000dil
Dilution ratio of anti-CRP antibody

FIG.12

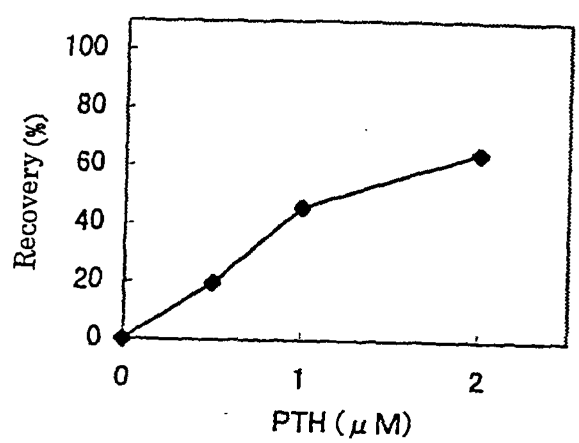
100
80
60
40
20
0
Recovery (%)
0
1
2
PTH (μM)

FIG.13

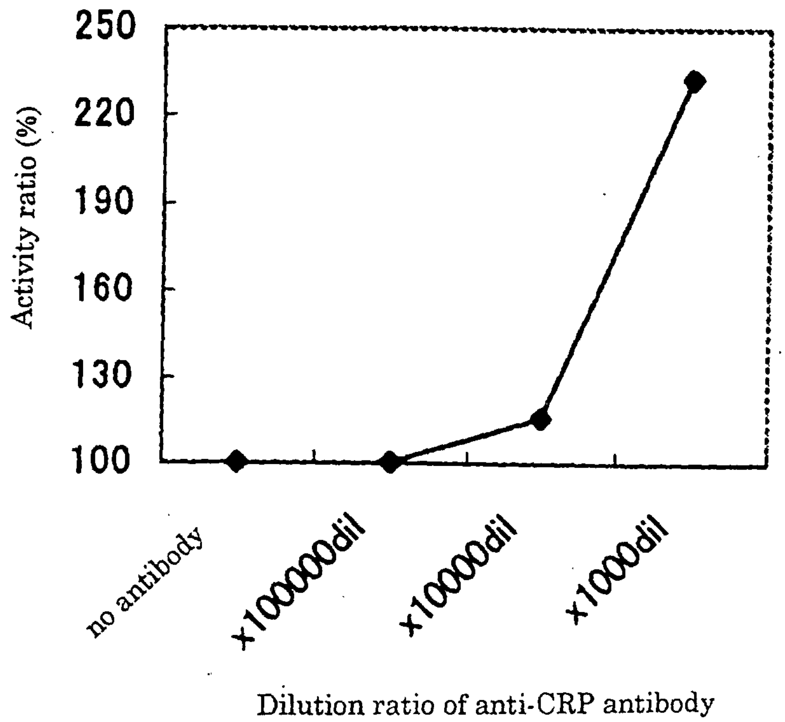
250
220
190
160
130
100
Activity ratio (%)
no antibody
x100000dil
x10000dil
x1000dil
Dilution ratio of anti-CRP antibody

FIG.14

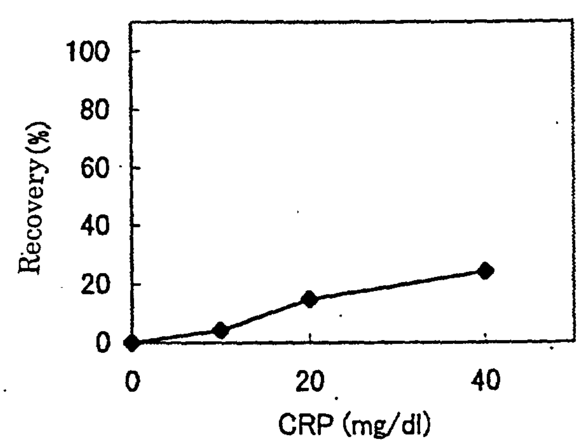
100
80
60
40
20
0
Recovery (%)
0
20
40
CRP (mg/dl)

European Patent Office

# EUROPEAN SEARCH REPORT

Application Number
EP 01 11 3996

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| X | WO 94 20636 A (ABBOTT LAB) 15 September 1994 (1994-09-15) * the whole document * --- | 1-46 | C07K19/00 C07K14/47 C12N9/04 C12N9/16 C12N9/38 C12N15/62 G01N33/50 |
| X | WO 86 06742 A (CALIFORNIA BIOTECHNOLOGY INC) 20 November 1986 (1986-11-20) * the whole document * --- | 1-46 | |
| X,D | FELIU J X ET AL: "Distinct mechanisms of antibody-mediated enzymatic reactivation in beta-galactosidase molecular sensors" FEBS LETTERS, ELSEVIER SCIENCE PUBLISHERS, AMSTERDAM, NL, vol. 438, no. 3, 6 November 1998 (1998-11-06), pages 267-271, XP004258604 ISSN: 0014-5793 * the whole document * --- | 1-46 | |
| X | BENITO ANTONI ET AL: "Beta-Galactosidase enzymatic activity as a molecular probe to detect specific antibodies." JOURNAL OF BIOLOGICAL CHEMISTRY, vol. 271, no. 35, 1996, pages 21251-21256, XP002177003 ISSN: 0021-9258 * the whole document * --- | 1-46 | TECHNICAL FIELDS SEARCHED (Int.Cl.7) C12N C07K G01N |
| X | LEGENDRE DANIEL ET AL: "Engineering a regulatable enzyme for homogeneous immunoassays." NATURE BIOTECHNOLOGY, vol. 17, no. 1, January 1999 (1999-01), pages 67-72, XP002177004 ISSN: 1087-0156 * the whole document * --- -/-- | 1-46 | |

The present search report has been drawn up for all claims

| Place of search | Date of completion of the search | Examiner |
|---|---|---|
| MUNICH | 7 September 2001 | Puonti-Kaerlas, J |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

European Patent Office

# EUROPEAN SEARCH REPORT

Application Number
EP 01 11 3996

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| X | US 5 534 223 A (BOQUET PAUL ET AL) 9 July 1996 (1996-07-09) * the whole document * | 1-46 | |
| X | EP 0 242 243 A (CENTRE NAT RECH SCIENT ;PASTEUR INSTITUT (FR)) 21 October 1987 (1987-10-21) * the whole document * | 1-46 | |
| P,X | FERRER-MIRALLES NEUS ET AL: "Molecular mechanisms for antibody-mediated modulation of peptide-displaying enzyme sensors." BIOCHEMICAL AND BIOPHYSICAL RESEARCH COMMUNICATIONS, vol. 275, no. 2, 28 August 2000 (2000-08-28), pages 360-364, XP002177005 ISSN: 0006-291X * the whole document * | 1-46 | |
| | | | TECHNICAL FIELDS SEARCHED (Int.Cl.7) |
| Y | US 4 959 323 A (ACS GEORGE ET AL) 25 September 1990 (1990-09-25) * the whole document * | 1-46 | |
| Y | US 6 033 890 A (BARNETT CHRISTOPHER CHARLES ET AL) 7 March 2000 (2000-03-07) * the whole document * | 1-46 | |
| Y | WO 91 00872 A (RUSH PRESBYTERIAN ST LUKE) 24 January 1991 (1991-01-24) * the whole document * | 1-46 | |

The present search report has been drawn up for all claims

| Place of search | Date of completion of the search | Examiner |
|---|---|---|
| MUNICH | 7 September 2001 | Puonti-Kaerlas, J |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT ON EUROPEAN PATENT APPLICATION NO. EP 01 11 3996

This annex lists the patent family members relating to the patent documents cited in the above-mentioned European search report.
The members are as contained in the European Patent Office EDP file on
The European Patent Office is in no way liable for these particulars which are merely given for the purpose of information.

07-09-2001

| Patent document cited in search report | Publication date | Patent family member(s) | Publication date |
|---|---|---|---|
| WO 9420636 A | 15-09-1994 | AU 6550294 A | 26-09-1994 |
| | | EP 0688364 A | 27-12-1995 |
| | | JP 8507686 T | 20-08-1996 |
| | | US 5843634 A | 01-12-1998 |
| WO 8606742 A | 20-11-1986 | EP 0222876 A | 27-05-1987 |
| | | US 4745055 A | 17-05-1988 |
| US 5534223 A | 09-07-1996 | FR 2648713 A | 28-12-1990 |
| | | US 5362644 A | 08-11-1994 |
| | | AT 133202 T | 15-02-1996 |
| | | CA 2019665 A | 26-12-1990 |
| | | DE 69024864 D | 29-02-1996 |
| | | DE 69024864 T | 09-05-1996 |
| | | EP 0407259 A | 09-01-1991 |
| | | JP 2815222 B | 27-10-1998 |
| | | JP 4004872 A | 09-01-1992 |
| EP 0242243 A | 21-10-1987 | FR 2595374 A | 11-09-1987 |
| | | AT 90968 T | 15-07-1993 |
| | | CA 1324333 A | 16-11-1993 |
| | | DE 3786294 A | 29-07-1993 |
| | | DE 3786294 T | 30-09-1993 |
| | | DK 116487 A | 08-09-1987 |
| | | JP 62282589 A | 08-12-1987 |
| | | PT 84423 A,B | 01-04-1987 |
| US 4959323 A | 25-09-1990 | NONE | |
| US 6033890 A | 07-03-2000 | AT 186598 T | 15-11-1999 |
| | | CA 2160115 A | 27-10-1994 |
| | | DE 69421617 D | 16-12-1999 |
| | | DE 69421617 T | 02-03-2000 |
| | | EP 0710360 A | 08-05-1996 |
| | | ES 2139087 T | 01-02-2000 |
| | | JP 8510638 T | 12-11-1996 |
| | | WO 9424559 A | 27-10-1994 |
| | | US 6090567 A | 18-07-2000 |
| WO 9100872 A | 24-01-1991 | US 5272258 A | 21-12-1993 |
| | | AU 642430 B | 21-10-1993 |
| | | AU 6352490 A | 06-02-1991 |
| | | CA 2057058 A | 28-12-1990 |
| | | EP 0479922 A | 15-04-1992 |
| | | JP 4505857 T | 15-10-1992 |

EPO FORM P0459

For more details about this annex : see Official Journal of the European Patent Office, No. 12/82

SEQUENCE LISTING

<110> WAKO PURE CHEMICAL INDUSTRIES, LTD.

<120> Hybrid Enzymes and Use Thereof

<130> WJ018

<140>

<141>

<160> 56

<170> PatentIn Ver. 2.1

<210> 1

<211> 206

<212> PRT

<213> Human

<400> 1

Gln Thr Asp Met Ser Arg Lys Ala Phe Val Phe Pro Lys Glu Ser Asp

1 5 10 15

Thr Ser Tyr Val Ser Leu Lys Ala Pro Leu Thr Lys Pro Leu Lys Ala

20 25 30

Phe Thr Val Cys Leu His Phe Tyr Thr Glu Leu Ser Ser Thr Arg Gly
35 40 45

Tyr Ser Ile Phe Ser Tyr Ala Thr Lys Arg Gln Asp Asn Glu Ile Leu
50 55 60

Ile Phe Trp Ser Lys Asp Ile Gly Tyr Ser Phe Thr Val Gly Gly Ser
65 70 75 80

Glu Ile Leu Phe Glu Val Pro Glu Val Thr Val Ala Pro Val His Ile
85 90 95

Cys Thr Ser Trp Glu Ser Ala Ser Gly Ile Val Glu Phe Trp Val Asp
100 105 110

Gly Lys Pro Arg Val Arg Lys Ser Leu Lys Lys Gly Tyr Thr Val Gly
115 120 125

Ala Glu Ala Ser Ile Ile Leu Gly Gln Glu Gln Asp Ser Phe Gly Gly
130 135 140

Asn Phe Glu Gly Ser Gln Ser Leu Val Gly Asp Ile Gly Asn Val Asn
145 150 155 160

Met Trp Asp Phe Val Leu Ser Pro Asp Glu Ile Asn Thr Ile Tyr Leu
165 170 175

Gly Gly Pro Phe Ser Pro Asn Val Leu Asn Trp Arg Ala Leu Lys Tyr

180 185 190

Glu Val Gln Gly Glu Val Phe Thr Lys Pro Gln Leu Trp Pro

195 200 205

<210> 2

<211> 16

<212> PRT

<213> Human

<400> 2

Asp Met Ser Arg Lys Ala Phe Val Phe Pro Lys Glu Ser Asp Thr Ser

1 5 10 15

<210> 3

<211> 27

<212> PRT

<213> Human

<400> 3

Leu Val Gly Asp Ile Gly Asn Val Asn Met Trp Asp Phe Val Leu Ser

1 5 10 15

Pro Asp Glu Ile Asn Thr Ile Tyr Leu Gly Gly

20 25

<210> 4

<211> 12

<212> PRT

<213> Human

<400> 4

Leu Lys Lys Gly Tyr Thr Val Gly Ala Glu Ala Ser

1 5 10

<210> 5

<211> 10

<212> PRT

<213> Human

<400> 5

Arg Ala Leu Lys Tyr Glu Val Gln Gly Glu

1 5 10

<210> 6

<211> 486

<212> PRT

<213> Leuconostoc mesenteroides

<400> 6

Met Val Ser Glu Ile Lys Thr Leu Val Thr Phe Phe Gly Gly Thr Gly
1 5 10 15

Asp Leu Ala Lys Arg Lys Leu Tyr Pro Ser Val Phe Asn Leu Tyr Lys
20 25 30

Lys Gly Tyr Leu Gln Lys His Phe Ala Ile Val Gly Thr Ala Arg Gln
35 40 45

Ala Leu Asn Asp Asp Glu Phe Lys Gln Leu Val Arg Asp Ser Ile Lys
50 55 60

Asp Phe Thr Asp Asp Gln Ala Gln Ala Glu Ala Phe Ile Glu His Phe
65 70 75 80

Ser Tyr Arg Ala His Asp Val Thr Asp Ala Ala Ser Tyr Ala Val Leu
85 90 95

Lys Glu Ala Ile Glu Glu Ala Ala Asp Lys Phe Asp Ile Asp Gly Asn
100 105 110

Arg Ile Phe Tyr Met Ser Val Ala Pro Arg Phe Phe Gly Thr Ile Ala
115 120 125

Lys Tyr Leu Lys Ser Glu Gly Leu Leu Ala Asp Thr Gly Tyr Asn Arg

130 135 140

Leu Met Ile Glu Lys Pro Phe Gly Thr Ser Tyr Asp Thr Ala Ala Glu
145 150 155 160

Leu Gln Asn Asp Leu Glu Asn Ala Phe Asp Asp Asn Gln Leu Phe Arg
165 170 175

Ile Asp His Tyr Leu Gly Lys Glu Met Val Gln Asn Ile Ala Ala Leu
180 185 190

Arg Phe Gly Asn Pro Ile Phe Asp Ala Ala Trp Asn Lys Asp Tyr Ile
195 200 205

Lys Asn Val Gln Val Thr Leu Ser Glu Val Leu Gly Val Glu Glu Arg
210 215 220

Ala Gly Tyr Tyr Asp Thr Ala Gly Ala Leu Leu Asp Met Ile Gln Asn
225 230 235 240

His Thr Met Gln Ile Val Gly Trp Leu Ala Met Glu Lys Pro Glu Ser
245 250 255

Phe Thr Asp Lys Asp Ile Arg Ala Ala Lys Asn Ala Ala Phe Asn Ala
260 265 270

Leu Lys Ile Tyr Asp Glu Ala Glu Val Asn Lys Tyr Phe Val Arg Ala

275 280 285

Gln Tyr Gly Ala Gly Asp Ser Ala Asp Phe Lys Pro Tyr Leu Glu Glu

290 295 300

Leu Asp Val Pro Ala Asp Ser Lys Asn Asn Thr Phe Ile Ala Gly Glu

305 310 315 320

Leu Gln Phe Asp Leu Pro Arg Trp Glu Gly Val Pro Phe Tyr Val Arg

325 330 335

Ser Gly Lys Arg Leu Ala Ala Lys Gln Thr Arg Val Asp Ile Val Phe

340 345 350

Lys Ala Gly Thr Phe Asn Phe Gly Ser Glu Gln Glu Ala Gln Glu Ala

355 360 365

Val Leu Ser Ile Ile Ile Asp Pro Lys Gly Ala Ile Glu Leu Lys Leu

370 375 380

Asn Ala Lys Ser Val Glu Asp Ala Phe Asn Thr Arg Thr Ile Asp Leu

385 390 395 400

Gly Trp Thr Val Ser Asp Glu Asp Lys Lys Asn Thr Pro Glu Pro Tyr

405 410 415

Glu Arg Met Ile His Asp Thr Met Asn Gly Asp Gly Ser Asn Phe Ala

420 425 430

Asp Trp Asn Gly Val Ser Ile Ala Trp Lys Phe Val Asp Ala Ile Ser

435 440 445

Ala Val Tyr Thr Ala Asp Lys Ala Pro Leu Glu Thr Tyr Lys Ser Gly

450 455 460

Ser Met Gly Pro Glu Ala Ser Asp Lys Leu Leu Ala Ala Asn Gly Asp

465 470 475 480

Ala Trp Val Phe Lys Gly

485

<210> 7

<211> 39

<212> DNA

<213> Artificial Sequence

<220>

<223> Description of Artificial Sequence: Oligonucleotide primer

<400> 7

ataaggggta caccatggtt tcagaaatca agacgttag 39

<210> 8

<211> 30

<212> DNA

<213> Artificial Sequence

<220>

<223> Description of Artificial Sequence: Oligonucleotide primer

<400> 8

ttcccgggct ttaattaacc tttaaacacc 30

<210> 9

<211> 30

<212> DNA

<213> Artificial Sequence

<220>

<223> Description of Artificial Sequence: Oligonucleotide primer

<400> 9

tggttggtta gctatggaaa aaccagaatc 30

<210> 10

<211> 34

<212> DNA

<213> Artificial Sequence

<220>

<223> Description of Artificial Sequence: Oligonucleotide primer

<400> 10

taggatccag gtacgtctaa ttcttcaagg tatg 34

<210> 11

<211> 32

<212> DNA

<213> Artificial Sequence

<220>

<223> Description of Artificial Sequence: Oligonucleotide primer

<400> 11

atggatccgc tgattctaaa aacaatacct tc 32

<210> 12

<211> 25

<212> DNA

<213> Artificial Sequence

<220>

<223> Description of Artificial Sequence: Oligonucleotide primer

<400> 12

aagcttgcat gcctgcaggt tcccg 25

<210> 13

<211> 54

<212> DNA

<213> Artificial Sequence

<220>

<223> Description of Artificial Sequence: Oligonucleotides consisting of the DNA coding for amino acids of Sequence 2, and a partial restriction site of BamHI consisting of 5'end of "gatcc" and 3'end of "g".

<400> 13

gatccgacat gtcgaggaag gcttttgtgt ttcccaaaga gtcggatact tccg 54

<210> 14

<211> 54

<212> DNA

<213> Artificial Sequence

<220>

<223> Description of Artificial Sequence: Complemental DNA of Sequence 13

<400> 14

gatccggaag tatccgactc tttgggaaac acaaaagcct tcctcgacat gtcg 54

<210> 15

<211> 48

<212> DNA

<213> Artificial Sequence

<220>

<223> Description of Artificial Sequence: Oligonucleotide consisting of the DNA coding for partial amino acids of Sequence 3, and a partial restriction site of BamHI consisting of 5' end of "gatcc" and 3' end of "g".

<400> 15

gatccgtgct gtcaccagat gagattaaca ccatctatct tggcgggg 48

<210> 16

<211> 48

<212> DNA

<213> Artificial Sequence

<220>

<223> Description of Artificial Sequence: Complemental DNA of Sequence 15

<400> 16

gatccccgc caagatagat ggtgttaatc tcatctggtg acagcacg 48

<210> 17

<211> 42

<212> DNA

<213> Artificial Sequence

<220>

<223> Description of Artificial Sequence: Oligonucleotide consisting of the DNA coding for amino acids of Sequence 4, and a partial restriction site of BamHI consisting of 5'end of "gatcc" and 3'end of "g".

<400> 17

gatccctgaa gaagggatac actgtggggg cagaagcaag cg 42

<210> 18

<211> 42

<212> DNA

<213> Artificial Sequence

<220>

<223> Description of Artificial Sequence: Complemental DNA of Sequence 17

<400> 18

gatccgcttg cttctgcccc cacagtgtat cccttcttca gg 42

<210> 19

<211> 36

<212> DNA

<213> Artificial Sequence

<220>

<223> Description of Artificial Sequence: Oligonucleotide consisting of the DNA coding for amino acids of Sequence 5, and a partial restriction site of BamHI consisting of 5'end of "gatcc" and 3'end of "g".

<400> 19

gatcccgggc actgaagtat gaagtgcaag gcgaag 36

<210> 20

<211> 36

<212> DNA

<213> Artificial Sequence

<220>

<223> Description of Artificial Sequence: Complemental DNA of Sequence 19

<400> 20

gatccttcgc cttgcacttc atacttcagt gcccgg 36

<210> 21

<211> 47

<212> DNA

<213> Artificial Sequence

<220>

<223> Description of Artificial Sequence: Oligonucleotide consisting of the DNA coding for partial amino acids of Sequence 3, and a partial restriction site of BamHI consisting of 5' end of "gatcc" and 3' end of "g".

<400> 21

gatcctagtg ggagacattg gaaatgtgaa catgtgggac tttgtgg 47

<210> 22

<211> 47

<212> DNA

<213> Artificial Sequence

<220>

<223> Description of Artificial Sequence: Complemental DNA of Sequence 21

<400> 22

gatcccacaa agtcccacat gttcacattt ccaatgtctc ccactag 47

<210> 23
<211> 33
<212> DNA
<213> Artificial Sequence

<220>
<223> Description of Artificial Sequence: Oligonucleotide Primer

<400> 23
taggatccgt ctaattcttc aaggtatggc ttg 33

<210> 24
<211> 34
<212> DNA
<213> Artificial Sequence

<220>
<223> Description of Artificial Sequence: Oligonucleotide Primer

<400> 24
aaggatccgt acctgctgat tctaaaaaca atac 34

<210> 25
<211> 32

<212> DNA

<213> Artificial Sequence

<220>

<400> 25

ttggatccag caggtacgtc taattcttca ag 32

<210> 26

<211> 33

<212> DNA

<213> Artificial Sequence

<220>

<223> Description of Artificial Sequence: Oligonucleotide Primer

<400> 26

taggatccga ttctaaaaac aataccttca tcg 33

<210> 27

<211> 34

<212> DNA

<213> Artificial Sequence

<220>

<223> Description of Artificial Sequence: Oligonucleotide Primer

<400> 27
gggtgtttaa aggtggatcc taattaaagc ccgg 34

<210> 28
<211> 32
<212> DNA
<213> Artificial Sequence

<220>
<223> Description of Artificial Sequence: Oligonucleotide Primer

<400> 28
taggatcctt ctgaaccaaa gttaaacgtg cc 32

<210> 29
<211> 32
<212> DNA
<213> Artificial Sequence

<220>
<223> Description of Artificial Sequence: Oligonucleotide Primer

<400> 29

atggatccca agaagcacaa gaagctgtct tg 32

<210> 30
<211> 1024
<212> PRT
<213> Escherichia coli

<400> 30

Met Thr Met Ile Thr Asp Ser Leu Ala Val Val Leu Gln Arg Arg Asp
1 5 10 15

Trp Glu Asn Pro Gly Val Thr Gln Leu Asn Arg Leu Ala Ala His Pro
20 25 30

Pro Phe Ala Ser Trp Arg Asn Ser Glu Glu Ala Arg Thr Asp Arg Pro
35 40 45

Ser Gln Gln Leu Arg Ser Leu Asn Gly Glu Trp Arg Phe Ala Trp Phe
50 55 60

Pro Ala Pro Glu Ala Val Pro Glu Ser Trp Leu Glu Cys Asp Leu Pro
65 70 75 80

Glu Ala Asp Thr Val Val Val Pro Ser Asn Trp Gln Met His Gly Tyr
85 90 95

Asp Ala Pro Ile Tyr Thr Asn Val Thr Tyr Pro Ile Thr Val Asn Pro
100 105 110

Pro Phe Val Pro Thr Glu Asn Pro Thr Gly Cys Tyr Ser Leu Thr Phe
115 120 125

Asn Val Asp Glu Ser Trp Leu Gln Glu Gly Gln Thr Arg Ile Ile Phe
130 135 140

Asp Gly Val Asn Ser Ala Phe His Leu Trp Cys Asn Gly Arg Trp Val
145 150 155 160

Gly Tyr Gly Gln Asp Ser Arg Leu Pro Ser Glu Phe Asp Leu Ser Ala
165 170 175

Phe Leu Arg Ala Gly Glu Asn Arg Leu Ala Val Met Val Leu Arg Trp
180 185 190

Ser Asp Gly Ser Tyr Leu Glu Asp Gln Asp Met Trp Arg Met Ser Gly
195 200 205

Ile Phe Arg Asp Val Ser Leu Leu His Lys Pro Thr Thr Gln Ile Ser
210 215 220

Asp Phe His Val Ala Thr Arg Phe Asn Asp Asp Phe Ser Arg Ala Val
225 230 235 240

Leu Glu Ala Glu Val Gln Met Cys Gly Glu Leu Arg Asp Tyr Leu Arg
245 250 255

Val Thr Val Ser Leu Trp Gln Gly Glu Thr Gln Val Ala Ser Gly Thr
260 265 270

Ala Pro Phe Gly Gly Glu Ile Ile Asp Glu Arg Gly Gly Tyr Ala Asp
275 280 285

Arg Val Thr Leu Arg Leu Asn Val Glu Asn Pro Lys Leu Trp Ser Ala
290 295 300

Glu Ile Pro Asn Leu Tyr Arg Ala Val Val Glu Leu His Thr Ala Asp
305 310 315 320

Gly Thr Leu Ile Glu Ala Glu Ala Cys Asp Val Gly Phe Arg Glu Val
325 330 335

Arg Ile Glu Asn Gly Leu Leu Leu Leu Asn Gly Lys Pro Leu Leu Ile
340 345 350

Arg Gly Val Asn Arg His Glu His His Pro Leu His Gly Gln Val Met
355 360 365

Asp Glu Gln Thr Met Val Gln Asp Ile Leu Leu Met Lys Gln Asn Asn
370 375 380

Phe Asn Ala Val Arg Cys Ser His Tyr Pro Asn His Pro Leu Trp Tyr
385 390 395 400

Thr Leu Cys Asp Arg Tyr Gly Leu Tyr Val Val Asp Glu Ala Asn Ile
405 410 415

Glu Thr His Gly Met Val Pro Met Asn Arg Leu Thr Asp Asp Pro Arg
420 425 430

Trp Leu Pro Ala Met Ser Glu Arg Val Thr Arg Met Val Gln Arg Asp
435 440 445

Arg Asn His Pro Ser Val Ile Ile Trp Ser Leu Gly Asn Glu Ser Gly
450 455 460

His Gly Ala Asn His Asp Ala Leu Tyr Arg Trp Ile Lys Ser Val Asp
465 470 475 480

Pro Ser Arg Pro Val Gln Tyr Glu Gly Gly Gly Ala Asp Thr Thr Ala
485 490 495

Thr Asp Ile Ile Cys Pro Met Tyr Ala Arg Val Asp Glu Asp Gln Pro
500 505 510

Phe Pro Ala Val Pro Lys Trp Ser Ile Lys Lys Trp Leu Ser Leu Pro
515 520 525

Gly Glu Thr Arg Pro Leu Ile Leu Cys Glu Tyr Ala His Ala Met Gly
530 535 540

Asn Ser Leu Gly Gly Phe Ala Lys Tyr Trp Gln Ala Phe Arg Gln Tyr
545 550 555 560

Pro Arg Leu Gln Gly Gly Phe Val Trp Asp Trp Val Asp Gln Ser Leu
565 570 575

Ile Lys Tyr Asp Glu Asn Gly Asn Pro Trp Ser Ala Tyr Gly Gly Asp
580 585 590

Phe Gly Asp Thr Pro Asn Asp Arg Gln Phe Cys Met Asn Gly Leu Val
595 600 605

Phe Ala Asp Arg Thr Pro His Pro Ala Leu Thr Glu Ala Lys His Gln
610 615 620

Gln Gln Phe Phe Gln Phe Arg Leu Ser Gly Gln Thr Ile Glu Val Thr
625 630 635 640

Ser Glu Tyr Leu Phe Arg His Ser Asp Asn Glu Leu Leu His Trp Met
645 650 655

Val Ala Leu Asp Gly Lys Pro Leu Ala Ser Gly Glu Val Pro Leu Asp
660 665 670

Val Ala Pro Gln Gly Lys Gln Leu Ile Glu Leu Pro Glu Leu Pro Gln
675 680 685

Pro Glu Ser Ala Gly Gln Leu Trp Leu Thr Val Arg Val Val Gln Pro
690 695 700

Asn Ala Thr Ala Trp Ser Glu Ala Gly His Ile Ser Ala Trp Gln Gln
705 710 715 720

Trp Arg Leu Ala Glu Asn Leu Ser Val Thr Leu Pro Ala Ala Ser His
725 730 735

Ala Ile Pro His Leu Thr Thr Ser Glu Met Asp Phe Cys Ile Glu Leu
740 745 750

Gly Asn Lys Arg Trp Gln Phe Asn Arg Gln Ser Gly Phe Leu Ser Gln
755 760 765

Met Trp Ile Gly Asp Lys Lys Gln Leu Leu Thr Pro Leu Arg Asp Gln
770 775 780

Phe Thr Arg Ala Pro Leu Asp Asn Asp Ile Gly Val Ser Glu Ala Thr
785 790 795 800

Arg Ile Asp Pro Asn Ala Trp Val Glu Arg Trp Lys Ala Ala Gly His
805 810 815

Tyr Gln Ala Glu Ala Ala Leu Leu Gln Cys Thr Ala Asp Thr Leu Ala
820 825 830

Asp Ala Val Leu Ile Thr Thr Ala His Ala Trp Gln His Gln Gly Lys
835 840 845

Thr Leu Phe Ile Ser Arg Lys Thr Tyr Arg Ile Asp Gly Ser Gly Gln
850 855 860

Met Ala Ile Thr Val Asp Val Glu Val Ala Ser Asp Thr Pro His Pro
865 870 875 880

Ala Arg Ile Gly Leu Asn Cys Gln Leu Ala Gln Val Ala Glu Arg Val
885 890 895

Asn Trp Leu Gly Leu Gly Pro Gln Glu Asn Tyr Pro Asp Arg Leu Thr
900 905 910

Ala Ala Cys Phe Asp Arg Trp Asp Leu Pro Leu Ser Asp Met Tyr Thr
915 920 925

Pro Tyr Val Phe Pro Ser Glu Asn Gly Leu Arg Cys Gly Thr Arg Glu
930 935 940

Leu Asn Tyr Gly Pro His Gln Trp Arg Gly Asp Phe Gln Phe Asn Ile
945 950 955 960

Ser Arg Tyr Ser Gln Gln Gln Leu Met Glu Thr Ser His Arg His Leu
965 970 975

Leu His Ala Glu Glu Gly Thr Trp Leu Asn Ile Asp Gly Phe His Met
980 985 990

Gly Ile Gly Gly Asp Asp Ser Trp Ser Pro Ser Val Ser Ala Glu Phe
995 1000 1005

Gln Leu Ser Ala Gly Arg Tyr His Tyr Gln Leu Val Trp Cys Gln Lys
1010 1015 1020

<210> 31
<211> 448
<212> PRT
<213> Escherichia coli

<400> 31
Thr Pro Glu Met Pro Val Leu Glu Asn Arg Ala Ala Gln Gly Asp Ile
1 5 10 15

Thr Ala Pro Gly Gly Ala Arg Arg Leu Thr Gly Asp Gln Thr Ala Ala
20 25 30

Leu Arg Asp Ser Leu Ser Asp Lys Pro Ala Lys Asn Ile Ile Leu Leu
35 40 45

Ile Gly Asp Gly Met Gly Asp Ser Glu Ile Thr Ala Ala Arg Asn Tyr
50 55 60

Ala Glu Gly Ala Gly Gly Phe Phe Lys Gly Ile Asp Ala Leu Pro Leu
65 70 75 80

Thr Gly Gln Tyr Thr His Tyr Ala Leu Asn Lys Lys Thr Gly Lys Pro
85 90 95

Asp Tyr Val Thr Asp Ser Ala Ala Ser Ala Thr Ala Trp Ser Thr Gly
100 105 110

Val Lys Thr Tyr Asn Gly Ala Leu Gly Val Asp Ile His Glu Lys Asp
115 120 125

His Pro Thr Ile Leu Glu Met Ala Lys Ala Ala Gly Leu Ala Thr Gly
130 135 140

Asn Val Ser Thr Ala Glu Leu Gln Asp Ala Thr Pro Ala Ala Leu Val
145 150 155 160

Ala His Val Thr Ser Arg Lys Cys Tyr Gly Pro Ser Ala Thr Ser Glu
165 170 175

Lys Cys Pro Gly Asn Ala Leu Glu Lys Gly Gly Lys Gly Ser Ile Thr
180 185 190

Glu Gln Leu Leu Asn Ala Arg Ala Asp Val Thr Leu Gly Gly Gly Ala
195 200 205

Lys Thr Phe Ala Glu Thr Ala Thr Ala Gly Glu Trp Gln Gly Lys Thr
210 215 220

Leu Arg Glu Gln Ala Gln Ala Arg Gly Tyr Gln Leu Val Ser Asp Ala
225 230 235 240

Ala Ser Leu Asn Ser Val Thr Glu Ala Asn Gln Gln Lys Pro Leu Leu
245 250 255

Gly Leu Phe Ala Asp Gly Asn Met Pro Val Arg Trp Leu Gly Pro Lys
260 265 270

Ala Thr Tyr His Gly Asn Ile Asp Lys Pro Ala Val Thr Cys Thr Pro
275 280 285

Asn Pro Gln Arg Asn Asp Ser Val Pro Thr Leu Ala Gln Met Thr Asp
290 295 300

Lys Ala Ile Glu Leu Leu Ser Lys Asn Glu Lys Gly Phe Phe Leu Gln
305 310 315 320

Val Glu Gly Ala Ser Ile Asp Lys Gln Asp His Ala Ala Asn Pro Cys
325 330 335

Gly Gln Ile Gly Glu Thr Val Asp Leu Asp Glu Ala Val Gln Arg Ala
340 345 350

Leu Glu Phe Ala Lys Lys Glu Gly Asn Thr Leu Val Ile Val Thr Ala
355 360 365

Asp His Ala His Ala Ser Gln Ile Val Ala Pro Asp Thr Lys Ala Pro
370 375 380

Gly Leu Thr Gln Ala Leu Asn Thr Lys Asp Gly Ala Val Met Val Met
385 390 395 400

Ser Tyr Gly Asn Ser Glu Glu Asp Ser Gln Glu His Thr Gly Gln Leu
405 410 415

Arg Ile Ala Ala Tyr Gly Pro His Ala Ala Asn Val Val Gly Leu Thr
420 425 430

Asp Gln Thr Asp Leu Phe Tyr Thr Met Lys Ala Ala Leu Gly Leu Lys
435 440 445

<210> 32

<211> 45

<212> DNA

<213> Artificial Sequence

<220>

<223> Description of Artificial Sequence: Oligonucleotides consisting of the DNA coding for partial amino acids of Sequence 2, and a partial restriction site of BamHI consisting of 5' end of "gatcc" and 3' end of "g".

<400> 32

gatccgacat gtcgaggaag gcttttgtgt ttcccaaaga gtcgg 45

<210> 33

<211> 45

<212> DNA

<213> Artificial Sequence

<220>

<223> Description of Artificial Sequence: Complemental DNA of Sequence 32

<400> 33

gatcccgact ctttgggaaa cacaaaagcc ttcctcgaca tgtcg 45

<210> 34

<211> 36

<212> DNA

<213> Artificial Sequence

<220>

<223> Description of Artificial Sequence: Oligonucleotides consisting of the DNA coding for partial amino acids of Sequence 2, and a partial restriction site of BamHI consisting of 5' end of "gatcc" and 3' end of "g".

<400> 34

gatccaggaa ggcttttgtg tttcccaaag agtcgg 36

<210> 35

<211> 36

<212> DNA

<213> Artificial Sequence

<220>

<223> Description of Artificial Sequence: Complemental DNA of Sequence 34

<400> 35

gatcccgact ctttgggaaa cacaaaagcc ttcctg 36

<210> 36

<211> 36

<212> DNA

<213> Artificial Sequence

<220>

<223> Description of Artificial Sequence: Oligonucleotide primer

<400> 36

cacaggaaac agaccatggg atccgtttca gaaatc 36

<210> 37

<211> 32

<212> DNA

<213> Artificial Sequence

<220>

<223> Description of Artificial Sequence: Oligonucleotide primer

<400> 37

ttggatccat caccggcacc atattgtgca cg 32

<210> 38

<211> 33

<212> DNA

<213> Artificial Sequence

<220>

<223> Description of Artificial Sequence: Oligonucleotide primer

<400> 38

aaggatcctc agctgacttc aagccatacc ttg 33

<210> 39

<211> 31

<212> DNA

<213> Artificial Sequence

<220>

<223> Description of Artificial Sequence: Oligonucleotide primer

<400> 39

aaggatccaa ggtatggctt gaagtcagct g 31

<210> 40

<211> 31

<212> DNA

<213> Artificial Sequence

<220>

<223> Description of Artificial Sequence: Oligonucleotide primer

<400> 40

aaggatccaa ggtatggctt gaagtcagct g 31

<210> 41

<211> 30

<212> DNA

<213> Artificial Sequence

<220>

<223> Description of Artificial Sequence: Oligonucleotide primer

<400> 41

ggtacgtata attcatcaag gtatggcttg 30

<210> 42

<211> 26

<212> DNA

<213> Artificial Sequence

<220>

<223> Description of Artificial Sequence: Oligonucleotide primer

<400> 42

tatacgtacc tgctgattct aaaaac 26

<210> 43

<211> 30

<212> DNA

<213> Artificial Sequence

<220>

<223> Description of Artificial Sequence: Oligonucleotides consisting of the DNA coding for partial amino acids of Sequence 2 .

<400> 43

aggaaggctt ttgtgtttcc caaagagtcg 30

<210> 44

<211> 30

<212> DNA

<213> Artificial Sequence

<220>

<223> Description of Artificial Sequence: Complemental DNA of Sequence 43

<400> 44

cgactctttg ggaaacacaa aagccttcct 30

<210> 45

<211> 55

<212> PRT

<213> Hepatitis B virus

<400> 45

Met Gln Trp Asn Ser Thr Ala Phe His Gln Ala Leu Gln Asp Pro Arg
1 5 10 15

Val Arg Gly Leu Tyr Phe Pro Ala Gly Gly Ser Ser Ser Gly Thr Val
20 25 30

Asn Pro Ala Pro Asn Ile Ala Ser His Ile Ser Ser Ile Ser Ala Arg
35 40 45

Thr Gly Asp Pro Val Thr Asn
50 55

<210> 46

<211> 12

<212> PRT

<213> Hepatitis B virus

<400> 46

Asp Pro Arg Val Arg Gly Leu Tyr Phe Pro Ala Gly
1 5 10

<210> 47

<211> 42

<212> DNA

<213> Artificial Sequence

<220>

<223> Description of Artificial Sequence: Oligonucleotides consisting of the DNA coding for amino acids of Sequence 46, and a partial restriction site of BamHI consisting of 5'end of ″gatcc″ and 3'end of ″g″.

<400> 47

gatccgaccc gcgtgttcgt ggtctgtatt tcccggctgg tg 42

<210> 48

<211> 42

<212> DNA

<213> Artificial Sequence

<220>

<223> Description of Artificial Sequence: Complemental DNA of Sequence 47

<400> 48

gatccaccag ccgggaaata cagaccacga acacgcgggt cc 42

<210> 49

<211> 84

<212> PRT

<213> Human

<400> 49

Ala Val Ser Glu Ile Gln Phe Met His Asn Leu Gly Lys His Leu Ser
1 5 10 15

Ser Met Glu Arg Val Glu Trp Leu Arg Lys Lys Leu Gln Asp Val His
20 25 30

Asn Phe Val Ala Leu Gly Ala Ser Ile Ala Tyr Arg Asp Gly Ser Ser
35 40 45

Gln Arg Pro Arg Lys Lys Glu Asp Asn Val Leu Val Glu Ser His Gln
50 55 60

Lys Ser Leu Gly Glu Ala Asp Lys Ala Asp Val Asp Val Leu Ile Lys
65 70 75 80

Ala Lys Pro Gln

<210> 50

<211> 15

<212> PRT

<213> Human

<400> 50

Glu Arg Val Glu Trp Leu Arg Lys Lys Leu Gln Asp Val His Asn

1 5 10 15

<210> 51

<211> 51

<212> DNA

<213> Artificial Sequence

<220>

<223> Description of Artificial Sequence: Oligonucleotides consisting of the DNA coding for amino acids of Sequence 50, and a partial restriction site of BamHI consisting of 5'end of "gatcc" and 3'end of "g".

<400> 51

gatccgaacg tgttgaatgg ctgcgtaaaa aactgcagga cgttcataac g 51

<210> 52

<211> 51

<212> DNA

<213> Artificial Sequence

<220>

<223> Description of Artificial Sequence: Complemental DNA of Sequence 51

<400> 52

gatccgttat gaacgtcctg cagtttttta cgcagccatt caacacgttc g 51

<210> 53

<211> 28

<212> DNA

<213> Artificial Sequence

<220>

<223> Description of Artificial Sequence: Oligonucleotide primer

<400> 53

tatgaccatg attacggatt cactggcc 28

<210> 54

<211> 26

<212> DNA

<213> Artificial Sequence

<220>

<223> Description of Artificial Sequence: Oligonucleotide primer

<400> 54

ctgcccggtt attattattt ttgacaccag 26

<210> 55

<211> 31

<212> DNA

<213> Artificial Sequence

<220>

<223> Description of Artificial Sequence: Oligonucleotide primer

<400> 55

taggatccta cgccaatgtc gttatccagc g 31

<210> 56

<211> 30

<212> DNA

<213> Artificial Sequence

<220>

<223> Description of Artificial Sequence: Oligonucleotide primer

<400> 56

ttggatccag tgaagcgacc cgcattgacc 30